(19) Europäisches Patentamt
European Patent Office
Office européen des brevets

(11) Veröffentlichungsnummer: **0 356 796 A2**

(12) # EUROPÄISCHE PATENTANMELDUNG

(21) Anmeldenummer: 89114958.5

(51) Int. Cl.⁵: **C07K 5/02 , A61K 37/64**

(22) Anmeldetag: 12.08.89

(30) Priorität: 27.08.88 DE 3829086
30.01.89 DE 3902615

(43) Veröffentlichungstag der Anmeldung:
07.03.90 Patentblatt 90/10

(84) Benannte Vertragsstaaten:
AT BE CH DE ES FR GB GR IT LI LU NL SE

(71) Anmelder: BAYER AG

D-5090 Leverkusen 1 Bayerwerk(DE)

(72) Erfinder: Henning, Rolf, Dr.
Böcklinstrasse 16
D-5600 Wuppertal 1(DE)
Erfinder: Benz, Günter, Dr.
Am Bölkumer Busch 5
D-5620 Velbert 15(DE)
Erfinder: Stasch, Johannes-Peter, Dr.
Schneewittchenweg 37
D-5600 Wuppertal 1(DE)
Erfinder: Knorr, Andreas, Dr.
Trillsergraben 10
D-4006 Erkrath/Hochdahl(DE)
Erfinder: Bender, Wolfgang, Dr.
Claudiusweg 5
D-5600 Wuppertal 1(DE)

(54) **Aminomethyl-peptide, Verfahren zur Herstellung und ihre Verwendung in Arzneimitteln.**

(57) Die Erfindung betrifft neue renininhibitorische Aminomethyl-peptide der allgemeinen Formel (I)

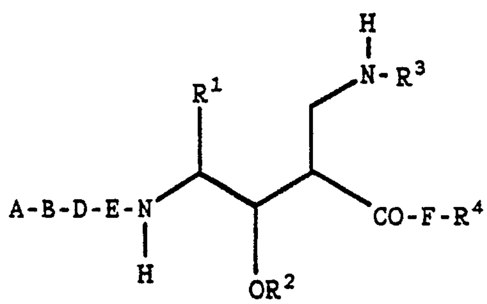

in welcher A, B, D, E, F, $R^1$, $R^2$, $R^3$ und $R^4$ die in der Beschreibung angegebene Bedeutung haben, Verfahren zu ihrer Herstellung und ihre Verwendung in Arzneimitteln, insbesondere in kreislaufbeeinflussenden Arzneimitteln.

EP 0 356 796 A2

## Aminomethyl-peptide, Verfahren zur Herstellung und ihre Verwendung in Arzneimitteln

Die Erfindung betrifft neue renininhibitorische Aminomethyl-peptide, Verfahren zu ihrer Herstellung und ihre Verwendung in Arzneimitteln, insbesondere in kreislaufbeeinflussenden Arzneimitteln.

Renin ist ein proteolytisches Enzym, das überwiegend von den Nieren produziert und ins Plasma sezerniert wird. Es ist bekannt, daß Renin in vivo vom Angiotensinogen das Dekapeptid Angiotensin I abspaltet. Angiotensin I wiederum wird in der Lunge, den Nieren oder anderen Geweben zum dem blutdruckwirksamen Oktapeptid Angiotensin II abgebaut. Die verschiedenen Effekte des Angiotensin II wie Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Die Aktivität des Renin-Angiotensin-Systems kann durch die Hemmung der Aktivität von Renin oder dem Angiotensin-Konversionsenzym (ACE) sowie durch Blockade von Angiotensin II-Rezeptoren pharmakologisch manipuliert werden. Die Entwicklung von oral einsetzbaren ACE-Hemmern hat somit zu neuen Antihypertensiva geführt (vgl. DOS 3 628 650, Am. J. Med. 77, 690, 1984).

Ein neuerer Ansatz ist, in die Renin-Angiotensin-Kaskade zu einem früheren Zeitpunkt einzugreifen, nämlich durch Inhibition der hochspezifischen Peptidase Renin.

Bisher wurden verschiedene Arten von Renininhibitoren entwickelt: Reninspezifische Antikörper, Phospholipide, Peptide mit der N-terminalen Sequenz des Prorenins, synthetische Peptide als Substratanaloga und modifizierte Peptide.

Es wurden nun neue Renininhibitoren gefunden, die sich von der Aminosäure (3S, 4S)-4-amino-3-hydroxy-6-methylheptancarbonsäure (Statin) ableiten (vgl. D.H. Rich, J. Med. Chem. 28, 263-73 (1985), Boger, J.; Lohr, N.S.; Ulm, E.H.; Poe, M.; Blaine, E.H.; Fanelli, G.M.; Lin, T.-Y.; Payne, L.S.; Schorn, T.W.; LaMont, B.I.; Vassil, T.C.; Stabilito, I.I.; Veber, D.F.; Rich, D.H.; Boparai, A.S., Nature (London) 1983, 303, 81.

Durch die Einführung einer Aminomethyl-Seitenkette zeigen sie neben einer hohen Selektivität gegenüber humanem Renin eine hohe Stabilität gegenüber enzymatischem Abbau und eine gute Wasserlöslichkeit. Die Erfindung betrifft Aminomethyl-peptide der allgemeinen Formel (I)

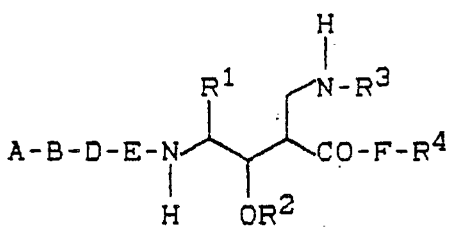

(I)

in welcher

A - Wasserstoff bedeutet oder

- für $C_1$-$C_8$-Alkyl, $C_1$-$C_8$-Alkylsulfonyl, Phenylsulfonyl oder Tolylsulfonyl steht oder
- für eine Gruppe der Formel $COR^5$ oder $COOR^6$ steht

worin

$R^5$ - für geradkettiges oder verzweigtes Alkyl mit bis zu 10 Kohlenstoffatomen steht, das gegebenenfalls durch Hydroxy, Aryl, Amino, Alkylamino mit bis zu 8 Kohlenstoffatomen je Alkylgruppe oder Dialkylamino mit bis zu 8 Kohlenstoffatomen je Alkylgruppe substituiert ist

und

$R^6$ - für geradkettiges oder verzweigtes Alkyl mit bis zu 8 Kohlenstoffatomen steht

oder

A- für eine Aminoschutzgruppe steht

B - für eine direkte Bindung steht oder

- für eine Gruppe der Formel

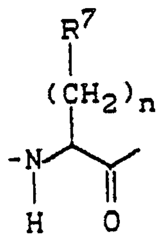 , 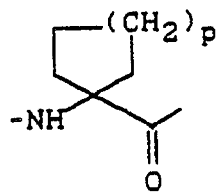 , 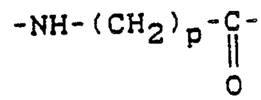

oder 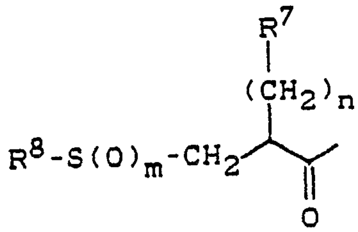 steht

worin
p - eine Zahl 1, 2 oder 3 bedeutet
m - eine Zahl 0, 1 oder 2 bedeutet
n - eine Zahl 0, 1, 2, 3 oder 4 bedeutet
$R^7$ - Wasserstoff, $C_1$-$C_8$-Alkyl, Hydroxymethyl, Hydroxyethyl, Carboxy, $C_1$-$C_8$-Alkoxycarbonyl oder Mercaptomethyl bedeutet oder
- für eine Gruppe der Formel $-CH_2-NH-R^8$ steht
worin
$R^8$ - für Wasserstoff, $C_1$-$C_8$-Alkyl, Phenylsulfonyl, $C_1$-$C_8$-Alkylsulfonyl oder
- für eine Aminoschutzgruppe steht
$R^7$ - Guanidinomethyl, Methylthiomethyl, Halogen, Indolyl, Imidazolyl, Pyridyl, Triazolyl oder Pyrazolyl bedeutet, das gegebenenfalls durch $R^8$ substituiert ist
wobei
$R^8$ die oben angegebene Bedeutung hat oder
- für Cycloalkyl mit 3 bis 8 Kohlenstoffatomen steht
- für Aryl steht, das bis zu 3-fach gleich oder verschieden substituiert sein kann durch $C_1$-$C_6$-Alkyl, $C_1$-$C_6$-Alkoxy, $C_1$-$C_6$-Alkylbenzyloxy, Trifluormethyl, Halogen, Hydroxy, Nitro oder durch eine Gruppe der Formel

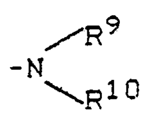

worin
$R^9$ und $R^{10}$ gleich oder verschieden sind und
- für Wasserstoff, $C_1$-$C_8$-Alkyl, $C_1$-$C_6$-Alkylsulfonyl, Aryl, Aralkyl, Tolylsulfonyl, Acetyl, Benzoyl oder
- für eine Aminoschutzgruppe stehen oder
B - für eine Gruppe der Formel

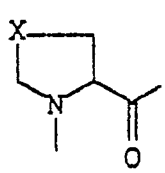 , 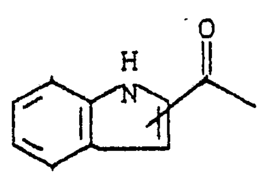 oder 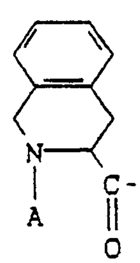 steht

worin
X - für Methylen, Hydroxymethylen, Ethylen, Schwefel oder Sauerstoff steht,
und
A die oben angegebene Bedeutung hat
D - die oben angegebene Bedeutung von B hat und mit dieser gleich oder verschieden ist,
E - die oben angegebene Bedeutung von B hat und mit dieser gleich oder verschieden ist,
F - die oben angegebene Bedeutung von B hat und mit dieser gleich oder verschieden ist,
$R^1$ - für geradkettiges oder verzweigtes Alkyl mit bis zu 10 Kohlenstoffatomen steht, das gegebenenfalls durch Halogen, Hydroxy, Cycloalkyl mit 3 bis 8 Kohlenstoffatomen, Amino, Alkylamino mit bis zu 8 Kohlenstoffatomen, Dialkylamino mit bis zu 8 Kohlenstoffatomen je Alkylgruppe oder Phenyl substituiert ist, welches seinerseits durch $C_1$-$C_8$-Alkyl, Amino, Nitro, Cyano oder Halogen substituiert sein kann oder
- für Aryl mit 6 bis 10 Kohlenstoffatomen steht, das bis zu 4-fach gleich oder verschieden durch $C_1$-$C_6$-Alkyl, $C_1$-$C_6$-Alkoxy, Hydroxy, Cyano, Nitro,Trifluormethyl, Trifluormethoxy, Trifluormethylthio, Phenyl oder durch die Gruppe der Formel

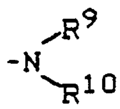

substituiert sein kann
worin
$R^9$ und $R^{10}$ gleich oder verschieden sind und die oben angegebene Bedeutung haben,
$R^2$ - Wasserstoff bedeutet oder
- für geradkettiges oder verzweigtes Alkyl mit bis zu 10 Kohlenwasserstoffatomen steht oder
- für die Gruppe der Formel
- $COR^5$ steht
worin
$R^5$ die oben angegebene Bedeutung hat,
$R^3$ - Wasserstoff bedeutet oder
- für geradkettiges oder verzweigtes Alkyl mit bis zu 10 Kohlenstoffatomen steht, das substituiert sein kann durch Halogen, Hydroxy, Aryl, Aralkyl oder Heteroaryl oder
- für eine Gruppe der Formel
-$COR^5$ steht
worin
$R^5$ die oben angegebene Bedeutung hat
$R^3$ - für Aryl steht, das bis zu 4-fach gleich oder verschieden substituiert sein kann durch Halogen, Hydroxy, Nitro, Cyano $C_1$-$C_8$-Alkoxy, $C_1$-$C_8$-Alkyl oder Amino
$R^4$ - für geradkettiges oder verzweigtes Alkyl mit bis zu 10 Kohlenstoffatomen steht, das gegebenenfalls durch Hydroxy, Cycloalkyl mit 3 bis 8 Kohlenstoffatomen, Halogen, $C_1$-$C_8$-Alkoxy, $C_1$-$C_8$-Alkoxycarbonyl oder Aryl substituiert ist oder
- für $C_1$-$C_8$-Alkoxy steht oder
- für Aryl steht, das bis zu 3-fach gleich oder verschieden durch Halogen, Hydroxy, Nitro, Cyano, Amino oder $C_1$-$C_8$-Alkoxy subtituiert sein kann oder
- für einen Rest
-HN-$R^{11}$ steht
worin
$R^{11}$ - Wasserstoff bedeutet oder
- für geradkettiges oder verzweigtes Alkyl mit bis zu 10 Kohlenstoffatomen steht, das gegebenenfalls durch Hydroxy, Halogen, $C_1$-$C_8$-Alkoxy, $C_1$-$C_8$-Alkoxycarbonyl, Cycloalkyl mit 3 bis 8 Kohlenstoffatomen, Aryl oder Heteroaryl substituiert ist oder
- für Cycloalkyl mit 3 bis 8 Kohlenstoffatomen steht oder
- für Phenyl steht, das durch Hydroxy, Halogen, Nitro, Cyano, $C_1$-$C_8$-Alkyl, $C_1$-$C_8$-Alkoxycarbonyl oder durch Amino substituiert sein kann,
und deren physiologisch unbedenklichen Salze.

Aminoschutzgruppe steht im Rahmen der Erfindung für die üblichen in der Peptid-Chemie verwendeten Aminoschutzgruppen.

Hierzu gehören bevorzugt: Benzyloxycarbonyl, 4-Brombenzyloxycarbonyl, 2-Chlorbenzyloxycarbonyl, 3-Chlorbenzyloxycarbonyl, Dichlorbenzyloxycarbonyl,3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Pentoxycarbonyl, Isopentoxycarbonyl, Hexoxycarbonyl, Cyclohexoxycarbonyl, Octoxycarbonyl, 2-Ethylhexoxycarbonyl, 2-Iodhexoxycarbonyl, 2-Bromethoxycarbonyl, 2-Chlorethoxycarbonyl, 2,2,2-Trichlorethoxycarbonyl, 2,2,2-Trichlor-tert-butoxycarbonyl, Benzhydryloxycarbonyl, Bis-(4-methoxyphenyl)methoxycarbonyl, Phenacyloxycarbonyl, 2-Trimethylsilylethoxycarbonyl, 2-(Di-n-butyl-methylsilyl)-ethoxycarbonyl, 2-Triphenylsilylethoxycarbonyl, 2-(Dimethyl-tert-butylsilyl)ethoxycarbonyl, Menthyloxycarbonyl, Vinyloxycarbonyl, Allyloxycarbonyl, Phenoxycarbonyl, Tolyloxycarbonyl, 2,4-Dinitrophenoxycarbonyl, 4-Nitrophenoxycarbonyl, 2,4,5-Trichlorphenoxycarbonyl, Naphthyloxycarbonyl, Fluorenyl-9-methoxycarbonyl, Ethylthiocarbonyl, Methylthiocarbonyl, Butylthiocarbonyl, Tert.-Butylthiocarbonyl, Phenylthiocarbonyl, Benzylthiocarbonyl, Methylaminocarbonyl, Ethylaminocarbonyl, Methylthiocarbonyl, Butylthiocarbonyl, tert-Butylthiocarbonyl, Phenylthiocarbonyl, Benzylthiocarbonyl, Methylaminocarbonyl, Ethylaminocarbonyl, Propylaminocarbonyl, iso-Propylaminocarbonyl, Formyl, Acetyl, Propionyl, Pivaloyl, 2-Chloracetyl, 2-Bromacetyl, 2-Iodacetyl, 2,2,2-Trifluoracetyl, 2,2,2-Trichloracetyl, Benzoyl, 4-Chlorbenzoyl, 4-Methoxybenzoyl, 4-Nitrobenzyl, 4-Nitrobenzoyl, Naphthylcarbonyl, Phenoxyacetyl, Adamantylcarbonyl, Dicyclohexylphosphoryl, Diphenylphosphoryl, Dibenzylphosphoryl, Di-(4-nitrobenzyl)phosphoryl, Phenoxyphenylphosphoryl, Diethylphosphinyl, Diphenylphosphinyl, Phthaloyl oder Phthalimido.

Besonders bevorzugte Aminoschutzgruppen sind Benzyloxycarbonyl, 3,4-Dimethoxybenzyloxycarbonyl, 3,5-Dimethoxybenzyloxycarbonyl, 4-Methoxybenzyloxycarbonyl, 4-Nitrobenzyloxycarbonyl, 2-Nitrobenzyloxycarbonyl, 3,4,5-Trimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert-Butoxycarbonyl, Cyclohexoxycarbonyl, Hexoxycarbonyl, Octoxycarbonyl, 2-Bromethoxycarbonyl, 2-Chlorethoxycarbonyl, Phenoxyacetyl, Naphthylcarbonyl, Adamantylcarbonyl, Phthaloyl, 2,2,2-Trichlorethoxycarbonyl, 2,2,2-Trichlor-tert- butoxycarbonyl, Menthyloxycarbonyl, Vinyloxycarbonyl, Allyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido oder Isovaleroyl.

Die erfindungsgemäßen Verbindungen der allgemeinen Formel (I), haben mehrere asymmetrische Kohlenstoffatome. Sie können unabhängig voneinander in der D- oder der L-Form vorliegen. Die Erfindung umfaßt die optischen Antipoden ebenso wie die Isomerengemische oder Racemate.

Bevorzugt liegen die Gruppen B, D, E und F unabhängig voneinander in der optisch reinen, bevorzugt in der L-Form vor.

Die Gruppe der Formel

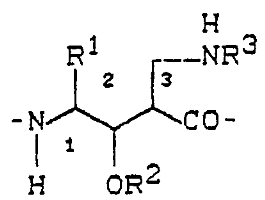

kann abhängig von der Definition der Reste bis zu 3 asymmetrische Kohlenstoffatome besitzen, die unabhängig voneinander in der R- oder S-Konfiguration vorliegen können. Bevorzugt liegt diese Gruppe in der 1S, 2S, 3R-Konfiguration, 1R, 2S, 3R-Konfiguration, 1S, 2R, 3S-Konfiguration oder in der 1R, 2R, 3S-Konfiguration vor.

Besonders bevorzugt sind die 1S, 2S, 3R-Konfiguration und die 1S, 2R, 3S-Konfiguration.

Die erfindungsgemäßen Verbindungen der allgemeinen Formel (I) können in Form ihrer Salze vorliegen. Dies können Salze der erfindungsgemäßen Verbindungen mit anorganischen oder organischen Säuren oder Basen sein. Zu den Säureadditionsprodukten gehören bevorzugt Salze mit Salzsäure, Bromwasserstoffsäure, Iodwasserstoffsäure, Schwefelsäure, Phosphorsäure oder mit Carbonsäuren wie Essigsäure, Propionsäure, Oxalsäure, Glykolsäure, Bernsteinsäure, Maleinsäure, Hydroxymaleinsäure, Methylmaleinsäure, Fumarsäure, Adipinsäure, Äpfelsäure, Weinsäure, Zitronensäure, Benzoesäure, Zimtsäure, Milchsäure, Ascorbinsäure, Salicylsäure, 2-Acetoxybenzoesäure, Nicotinsäure, Isonicotinsäure, oder Sulfonsäuren wie Methansulfonsäure, Ethansulfonsäure, Benzolsulfonsäure, Toluolsulfonsäure, Naphthalin-2-sulfonsäure oder Naphthalindisulfonsäure.

Bevorzugte Verbindungen der allgemeinen Formel (I) sind solche, in denen

A - Wasserstoff bedeutet oder

- für $C_1$-$C_6$-Alkyl, $C_1$-$C_6$-Alkylsulfonyl, Phenylsulfonyl oder Tolylsulfonyl steht oder

- für eine Gruppe der Formel $COR^5$ oder $COOR^6$ steht worin

$R^5$ für geradkettiges oder verzweigtes Alkyl mit bis zu 8 Kohlenstoffatomen steht, das gegebenenfalls durch Hydroxy, Phenyl, Amino, Alkylamino mit bis zu 6 Kohlenstoffatomen oder Dialkylamino mit bis zu 6 Kohlenstoffatomen je Alkylgruppe substituiert ist

und

$R^6$ - für geradkettiges oder verzweigtes Alkyl mit bis zu 6 Kohlenstoffatomen steht

oder

A - für eine Aminoschutzgruppe steht

B - für eine direkte Bindung steht oder

- für einen Rest der Formel

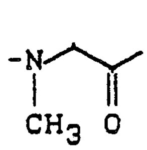 , 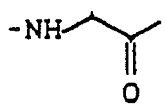 , 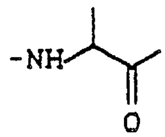 ,

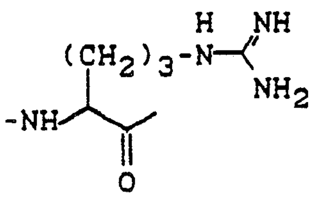 , 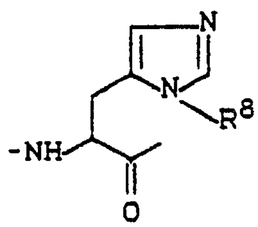 , 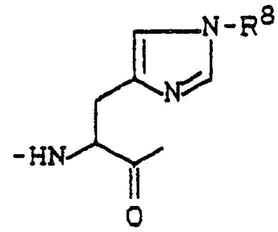

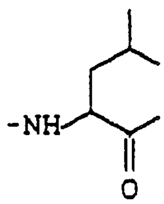 , 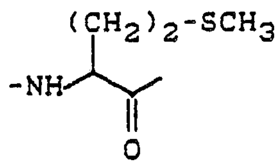 , 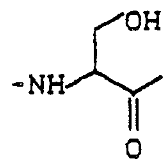

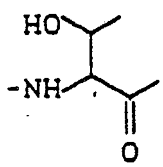 , 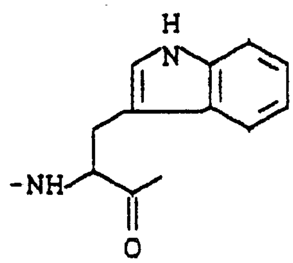 , 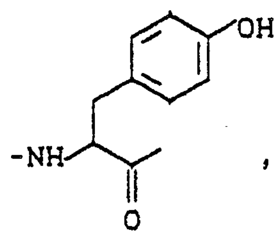 ,

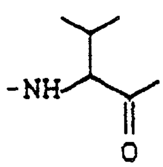 , 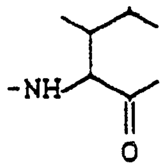 , 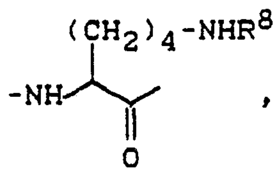 , 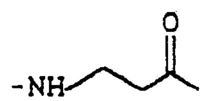 ,

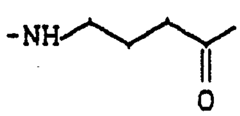 , 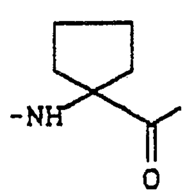 , 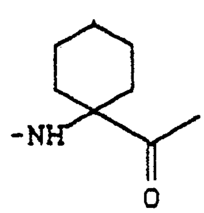

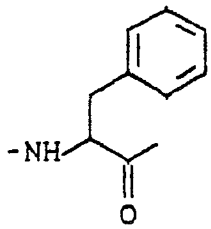 , 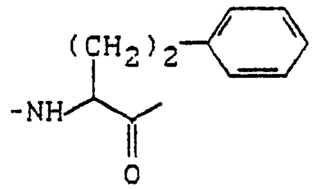 , 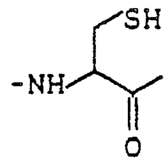 ,

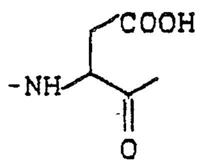 , 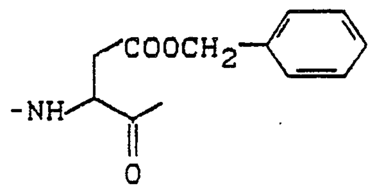 , 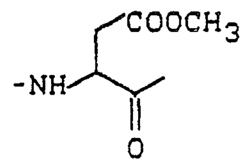 ,

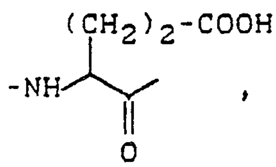 , 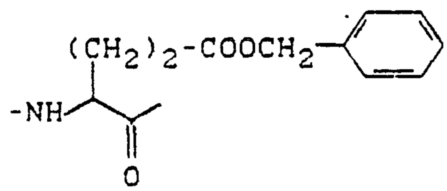 ,

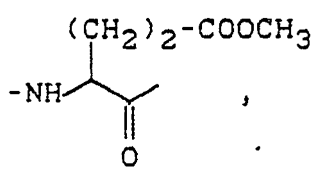 , 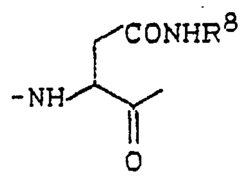 ,

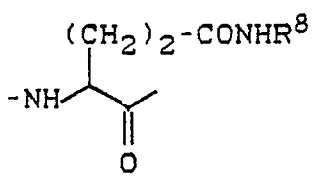 , 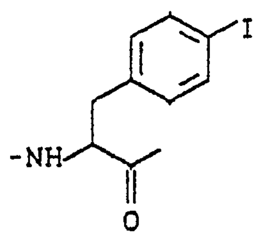 , 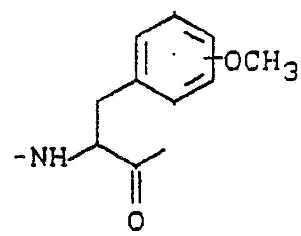 ,

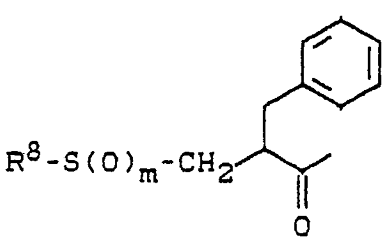 , 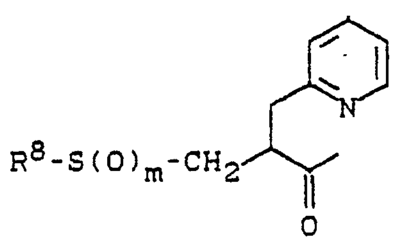 ,

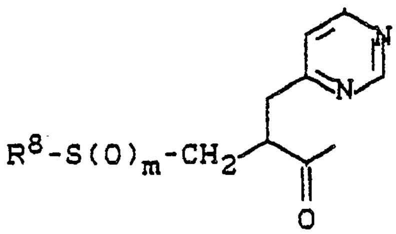
,
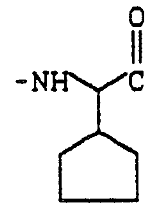
,
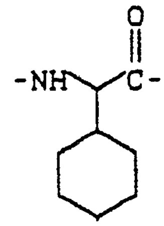
,

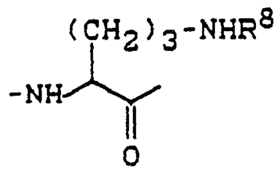
,
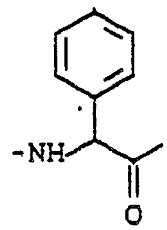
,
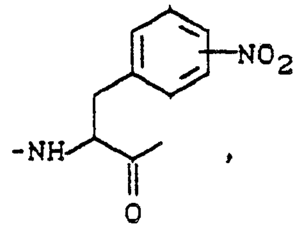
,

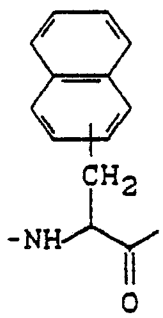
,
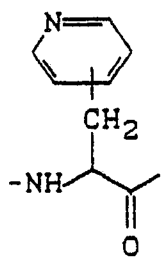
,
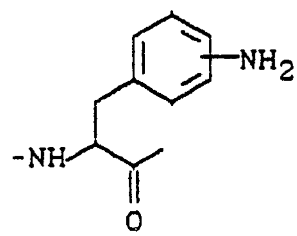

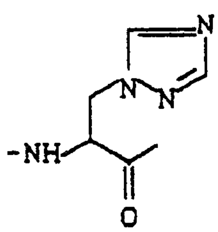
oder
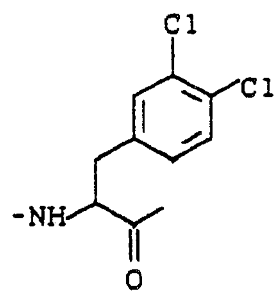

in ihrer D-Form, L-Form oder als D,L-Isomerengemisch, bevorzugt in der L-Form, worin

m - eine Zahl 0, 1 oder 2 bedeutet

$R^8$ - Wasserstoff bedeutet oder

- für $C_1$-$C_6$-Alkyl, Phenylsulfonyl, $C_1$-$C_4$-Alkylsulfonyl oder

- für eine Aminoschutzgruppe steht,

B - für eine Gruppe der Formel

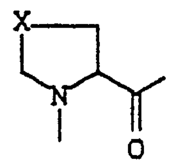 , 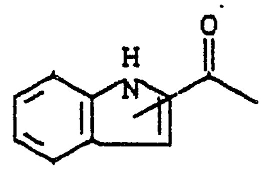 oder 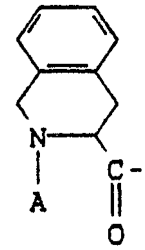 steht

worin
X - Methylen, Schwefel oder Sauerstoff bedeutet
und
A die oben angegebene Bedeutung hat
in ihrer L-Form, D-Form oder als D,L-Isomerengemisch,
D, E und F gleich oder verschieden sind und die gleiche Bedeutung wie B haben und mit dieser gleich oder verschieden sind
$R^1$ - für geradkettiges oder verzweigtes Alkyl mit bis zu 8 Kohlenstoffatomen steht, das gegebenenfalls durch Fluor, Chlor, Brom, Hydroxy, Cycloalkyl mit 3 bis 8 Kohlenstoffatomen, Amino, Alkylamino mit bis zu 6 Kohlenstoffatomen, Dialkylamino mit bis zu 6 Kohlenstoffatomen je Alkylgruppe oder Phenyl substituiert ist, welches seinerseits durch $C_1$-$C_6$-Alkyl, Amino, Nitro, Cyano oder Halogen substituiert sein kann oder
- für Phenyl steht, das bis zu 3-fach gleich oder verschieden durch $C_1$-$C_4$-Alkyl, $C_1$-$C_4$-Alkoxy, Hydroxy, Cyano, Nitro, Trifluormethyl, Trifluormethoxy, Trifluormethylthio, Phenyl oder durch eine Gruppe der Formel

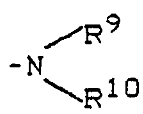

substituiert sein kann
worin
$R^9$ und $R^{10}$ gleich oder verschieden sind
und
- für Wasserstoff, $C_1$-$C_6$-Alkyl, $C_1$-$C_4$-Alkylsulfonyl, Phenyl, Benzyl, Tolylsulfonyl, Acetyl oder Benzoyl stehen oder
- eine Aminoschutzgruppe bedeuten,
$R^2$ - Wasserstoff bedeutet, oder
- für geradkettiges oder verzweigtes Alkyl mit bis zu 8 Kohlenstoffatomen steht oder
- für die Gruppe der Formel $COR^5$ steht,
worin
$R^5$ die oben angegebene Bedeutung hat,
$R^3$ - Wasserstoff bedeutet oder
- für geradkettiges oder verzweigtes Alkyl mit bis zu 8 Kohlenstoffatomen steht, das substituiert sein kann durch Fluor, Chlor, Brom, Hydroxy, Phenyl, Benzyl, Pyridyl oder Pyrimidyl oder
- für eine Gruppe der Formel $COR^5$ steht,
worin
$R^5$ die oben angegebene Bedeutung hat
oder
$R^3$ - für Phenyl steht, das bis zu 3-fach gleich oder verschieden substituiert sein kann durch Fluor, Chlor, Brom, Hydroxy, Nitro, Cyano, $C_1$-$C_4$-Alkoxy, $C_1$-$C_4$-Alkyl oder Amino
$R^4$ - für geradkettiges oder verzweigtes Alkyl mit bis zu 8 Kohlenstoffatomen steht, das gegebenenfalls durch Hydroxy, Cyclopropyl, Cyclopentyl, Cyclohexyl, Fluor, Chlor, Brom, $C_1$-$C_6$-Alkoxy, $C_1$-$C_6$-Alkoxycarbonyl oder Phenyl substituiert ist oder
- für $C_1$-$C_6$-Alkoxy steht oder
- für Phenyl steht, das bis zu 2-fach gleich oder verschieden durch Fluor, Chlor, Brom, Hydroxy, Nitro, Cyano, Amino oder $C_1$-$C_6$-Alkoxy substituiert sein kann oder
- für einen Rest

-HN-$R^{11}$ steht
worin
$R^{11}$ - Wasserstoff bedeutet oder
- für geradkettiges oder verzweigtes Alkyl mit bis zu 8 Kohlenstoffatomen steht, das gegebenenfalls durch Hydroxy, Fluor, Chlor, Brom, $C_1$-$C_6$-Alkoxy, $C_1$-$C_6$-Alkoxycarbonyl, Cyclopropyl, Cyclopentyl, Cyclohexyl, Phenyl, Pyridyl oder Pyrimidyl substituiert ist oder
- für Cyclopropyl, Cyclopentyl oder Cyclohexyl steht oder
- für Phenyl steht, das durch Hydroxy, Fluor,Chlor, Brom, Nitro, Cyano, $C_1$-$C_6$-Alkyl, $C_1$-$C_6$-Alkoxycarbonyl oder durch Amino substituiert sein kann,
und deren physiologisch unbedenklichen Salze.

Besonders bevorzugte Verbindungen der allgemeinen Formel (I) sind solche, in denen
A - Wasserstoff bedeutet oder
- für $C_1$-$C_4$-Alkyl, $C_1$-$C_4$-Alkylsulfonyl, Phenylsulfonyl oder Tolylsulfonyl steht oder
- eine Gruppe der Formel
-$COR^5$ oder -$COOR^6$ steht
worin
$R^5$ - für geradkettiges oder verzweigtes Alkyl mit bis zu 6 Kohlenstoffatomen steht, das gegebenenfalls durch Hydroxy, Phenyl, Amino, Alkylamino mit bis zu 4 Kohlenstoffatomen oder durch Dialkylamino mit bis zu 4 Kohlenstoffatomen je Alkylgruppe substituiert ist
$R^6$ - für geradkettiges oder verzweigtes Alkyl mit bis zu 4 Kohlenstoffatomen steht oder
A - für eine Aminoschutzgruppe steht,
B - für eine direkte Bindung steht oder
- für Glycyl (Gly), Alanyl (Ala), Arginyl (Arg), Histidyl (His), Leucyl (Leu), Isoleucyl (Ile), Seryl (Ser), Threonyl (Thr), Tryptophyl (Trp), Tyrosyl (Tyr), Valyl (Val), Lysyl (Lys), Asparagyl (Asp), Asparaginamido (Asn), Glutamyl (Glu), Glutaminamido (Gln), Cystyl (Cys), Methionyl (Met), Phenylalanyl (Phe), 2-, 3- oder 4-Nitrophenylalanyl, 2-, 3- oder 4-Aminophenylalanyl oder Pyridylalanyl, gegebenenfalls mit Aminoschutzgruppe, oder Prolyl (Pro) steht, wobei die Eiweißgruppen jeweils in ihrer L-Form oder D-Form vorliegen können
oder
- für eine Gruppe der Formel

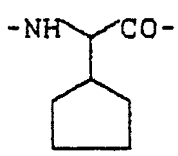 , 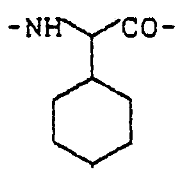 , 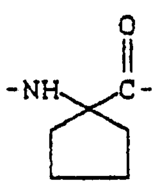 , 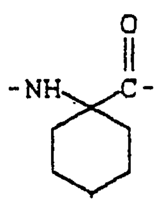 , 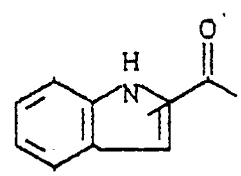 ,

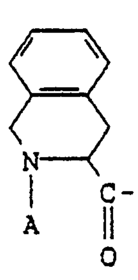 , 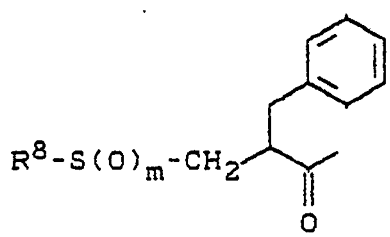 oder 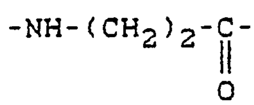 ,

steht

worin
A die oben angegebene Bedeutung hat und
$R^8$ für $C_1$-$C_4$-Alkyl Steht
D, E und F gleich oder verschieden sind und die gleiche Bedeutung wie B haben und mit dieser gleich oder verschieden sind,
$R^1$ - für geradkettiges oder verzweigtes Alkyl mit bis zu 6 Kohlenstoffatomen steht, das gegebenenfalls durch Fluor, Chlor, Hydroxy, Cyclopropyl, Cyclopentyl, Cyclohexyl, Amino, Alkylamino mit bis zu 4 Kohlenstoffatomen, Dialkylamino mit bis zu 4 Kohlenstoffatomen je Alkylgruppe, oder Phenyl substituiert ist, welches seinerseits durch $C_1$-$C_3$-Alkyl, Amino, Nitro, Cyano, Fluor oder Chlor substituiert sein kann oder

- für Phenyl steht, das bis zu 2-fach gleich oder verschieden durch $C_1$-$C_3$-Alkyl, $C_1$-$C_2$-Alkoxy, Hydroxy, Cyano, Nitro, Trifluormethyl, Trifluormethoxy, Trifluormethylthio, Phenyl oder durch eine Gruppe der Formel

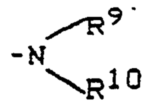

substituiert sein kann
worin
$R^9$ und $R^{10}$ gleich oder verschieden sind und
- für Wasserstoff, $C_1$-$C_3$-Alkyl, $C_1$-$C_3$-Alkylsulfonyl, Phenyl, Benzyl, Tolylsufonyl, Acetyl oder Benzoyl stehen oder
- eine Aminoschutzgruppe bedeuten,
$R^2$ Wasserstoff bedeutet oder
- für geradkettiges oder verzweigtes Alkyl mit bis zu 6 Kohlenstoffatomen steht oder
- für die Gruppe der Formel
$-COR^5$ steht
worin
$R^5$ die oben angegebene Bedeutung hat
$R^3$ - Wasserstoff bedeutet oder
- für geradkettiges oder verzweigtes Alkyl mit bis zu 6 Kohlenstoffatomen steht, das substituiert sein kann durch Fluor, Chlor, Hydroxy, Phenyl, Pyridyl oder Pyrimidyl oder
- für eine Gruppe der Formel
$-COR^5$ steht
worin
$R^5$ die oben angegebene Bedeutung hat oder
$R^3$ - für Phenyl steht, das bis zu 2-fach gleich oder verschieden substituiert sein kann durch Fluor, Chlor, Hydroxy, Nitro, Cyano, $C_1$-$C_2$-Alkoxy, $C_1$-$C_3$-Alkyl oder Amino
$R^4$ - für geradkettiges oder verzweigtes Alkyl mit bis zu 6 Kohlenstoffatomen steht, das gegebenenfalls durch Hydroxy, Cyclopropyl, Cyclopentyl, Cyclohexyl, Fluor, Chlor, $C_1$-$C_4$-Alkoxy, $C_1$-$C_4$-Alkoxycarbonyl oder Phenyl substituiert ist oder
- für $C_1$-$C_4$-Alkoxy oder
- für Phenyl steht, das durch Fluor, Chlor, Hydroxy, Nitro, Cyano, Amino oder $C_1$-$C_4$-Alkoxy substituiert sein kann
oder
- für einen Rest
$H-N-R^{11}$ steht
worin
$R^{11}$ - Wasserstoff bedeutet oder
- für geradkettiges oder verzweigtes Alkyl mit bis zu 6 Kohlenstoffatomen steht, das gegebenenfalls durch Hydroxy, Fluor, Chlor, Brom, $C_1$-$C_4$- Alkoxy, $C_1$-$C_4$-Alkoxycarbonyl, Cyclopropyl, Cyclopentyl, Cyclohexyl, Phenyl, Pyridyl oder Pyrimidyl substituiert ist oder
- für Cyclopropyl, Cyclopentyl oder Cyclohexyl steht oder
- für Phenyl steht, das durch Fluor, Chlor, Brom, Nitro, Cyano, $C_1$-$C_3$-Alkyl, $C_1$-$C_3$-Alkoxycarbonyl oder durch Amino substituiert sein kann,
sowie deren physiologisch unbedenkliche Salze.

Salze der erfindungsgemäßen Verbindungen mit salzbildenden Gruppen können in an sich bekannter Weise hergestellt werden, zum Beispiel durch Umsetzung der erfindungsgemäßen Verbindungen, die saure Gruppen enthalten, mit entsprechenden Basen oder durch Umsetzung der erfindungsgemäßen Verbindungen, die basische Gruppen enthalten mit entsprechenden Säuren, jeweils bevorzugt mit den oben aufgeführten Basen bzw. Säuren.

Stereoisomerengemische, insbesondere Diastereomerengemische, können in an sich bekannter Weise, z.B. durch fraktionierte Kristallisation oder Chromatographie in die einzelnen Isomere getrennt werden.

Racemate können in an sich bekannter Weise, z.B. durch Überführung der optischen Antipoden in Diastereomere gespalten werden.

Die erfindungsgemäßen Verbindungen der allgemeinen Formel (I)

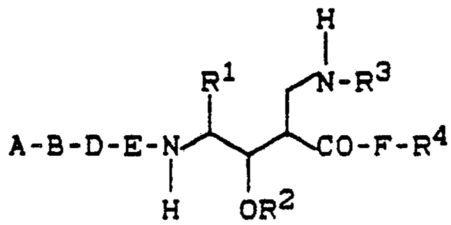

(I)

in welcher
A, B, D, E, F, $R^1$, $R^2$, $R^3$ und $R^4$ die oben angegebene Bedeutung haben,
erhält man, indem man
Verbindungen der allgemeinen Formel (II)

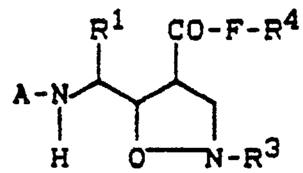

(II)

in welcher
$R^1$, $R^3$, $R^4$, A und F die oben angegebene Bedeutung haben,
zunächst deblockiert, indem die Gruppe A nach üblicher Methode abgespalten wird, und in einem zweiten Schritt mit Verbindungen der allgemeinen Formel (III)
A-B-D-E-OH (III)
in welcher
A, B, D und E die oben angegebene Bedeutung haben,
zu den Verbindungen der allgemeinen Formel (IIa)

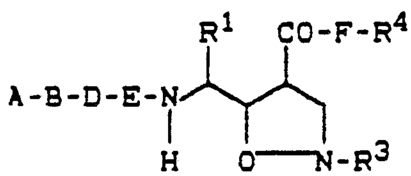

(IIa)

in welcher
A, B, D, E, F, $R^1$, $R^3$ und $R^4$ die oben angegebene Bedeutung haben,
umsetzt,
und die Verbindungen der allgemeinen Formel (IIa) anschließend durch Hydrogenolyse unter Ringöffnung reduziert.

Die Verbindungen der allgemeinen Formel (II) sind neu und können hergestellt werden, indem man Verbindungen der allgemeinen Formel (IIb)

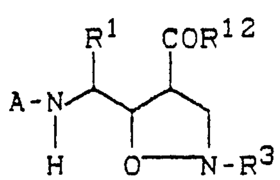

(IIb)

in welcher
A, $R^1$ und $R^3$ die oben angegebene Bedeutung haben und
$R^{12}$ - für $C_1$-$C_4$-Alkoxy steht,
zunächst nach üblichen Methoden verseift und anschießend mit Verbindungen der allgemeinen Formel (IV)
H-F-$R^4$ (IV)
in welcher

F und $R^4$ die oben angegebene Bedeutung haben,
umsetzt.

Die Verbindungen der allgemeinen Formel (IIb) sind neu und können hergestellt werden, indem man Verbindungen der allgemeinen Formel (V)

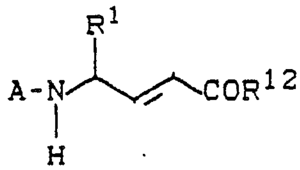

(V)

in welcher
A, $R^1$ und $R^{12}$ die oben angegebene Bedeutung haben,
mit Verbindungen der allgemeinen Formel (VI)

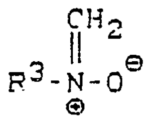

(VI)

in welcher
$R^3$ die oben angegebene Bedeutung hat,
in einer Nitron-Cycloadditionsreaktion umsetzt.

Die Synthese kann durch folgendes Reaktionschema beispielhaft belegt werden.

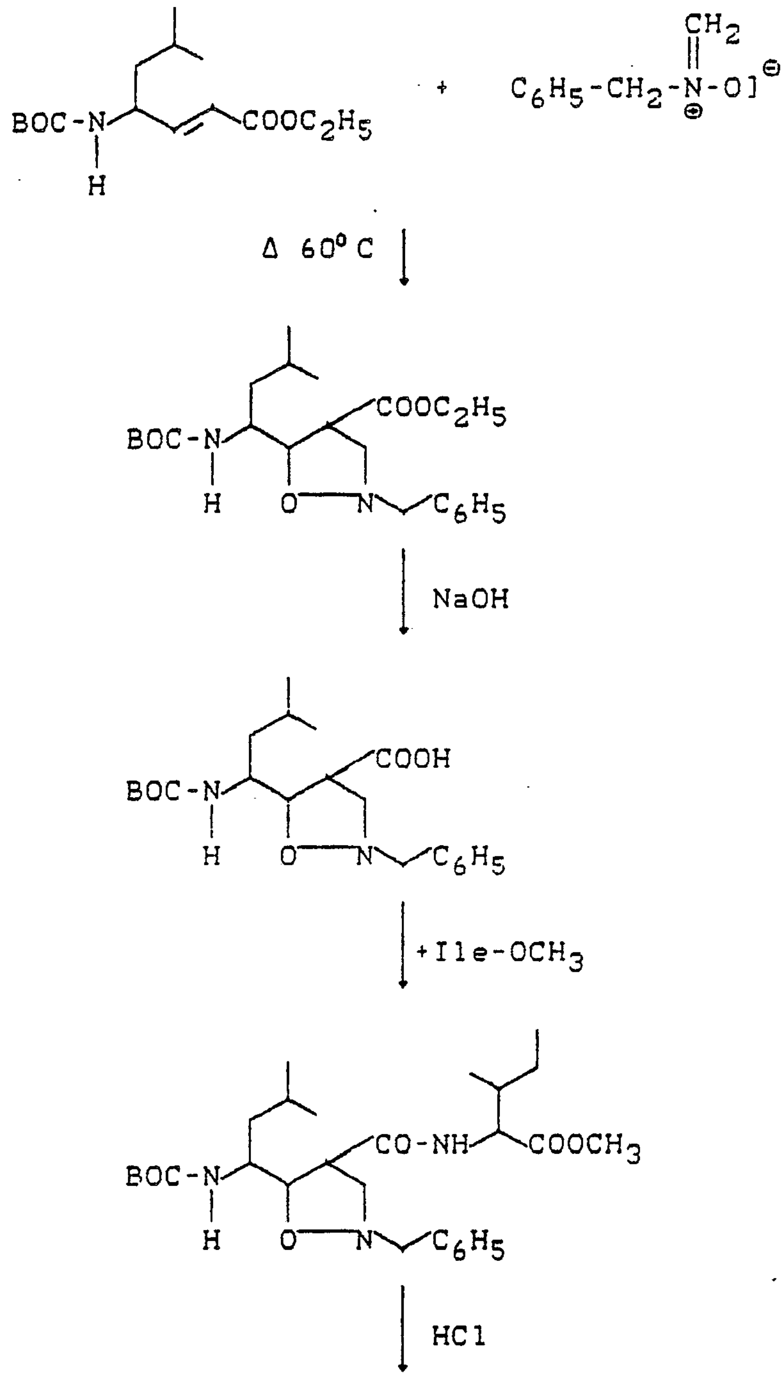
Δ 60° C
NaOH
+Ile-OCH3
HCl

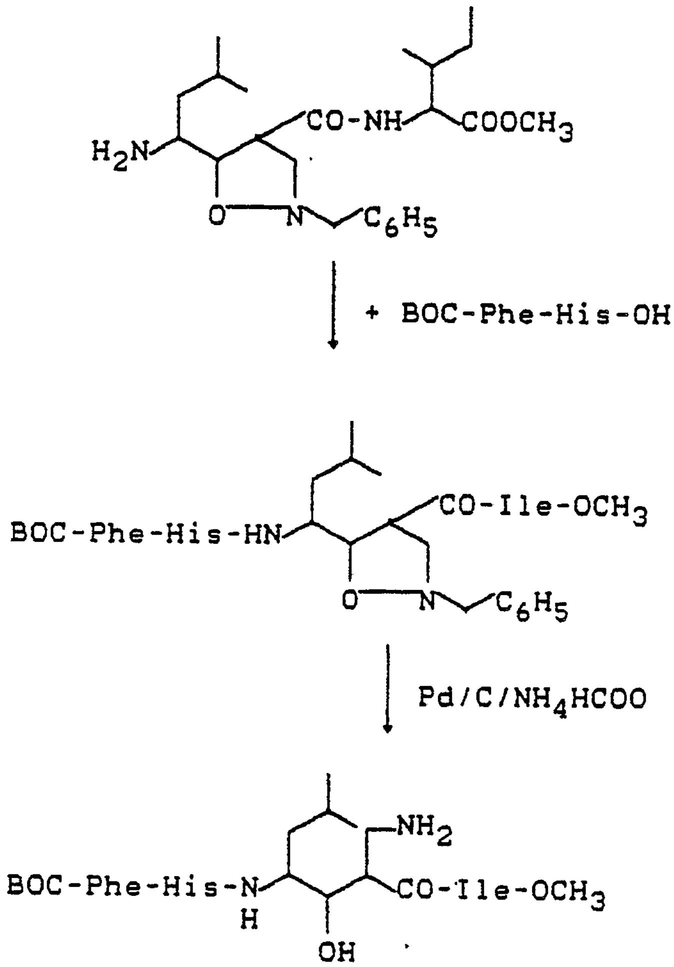


Als Lösemittel für die Additionen der Verbindungen der allgemeinen Formel (III) und (IV) eignen sich die üblichen inerten Lösemittel, die sich unter den jeweils gewählten Reaktionsbedingungen nicht verändern. Hierzu gehören Wasser oder organische Lösemittel wie Diethylether, Glykolmono- oder -dimethylether, Dioxan oder Tetrahydrofuran, oder Kohlenwasserstoffe wie Benzol, Toluol, Xylol, Cyclohexan oder Erdölfraktionen oder Halogenkohlenwasserstoffe wie Methylenchlorid, Chloroform, Tetrachlorkohlenwasserstoffe oder Aceton, Dimethylsulfoxid, Dimethylformamid, Hexamethylphosphorsäuretriamid, Essigester, Pyridin, Triethylamin oder Picolin.
Ebenso ist es möglich, Gemische der genannten Lösemittel zu verwenden.
Bevorzugt sind Tetrahydrofuran, Methylenchlorid, Dimethylformamid und Essigester.

Üblicherweise wird das Verfahren in Gegenwart geeigneter Löse- bzw.Verdünnungsmittel, gegebenenfalls in Anwesenheit eines Hilfsstoffes oder Katalysators in einem Temperaturbereich von -80 °C bis 300 °C, bevorzugt von -30 °C bis +30 °C bei normalem Druck durchgeführt. Ebenso ist es möglich, bei erhöhtem oder erniedrigtem Druck zu arbeiten.

Als Hilfsstoffe werden bevorzugt Kondensationsmittel eingesetzt, die auch Basen sein können, insbesondere wenn die Carboxylgruppe als Anhydrid aktiviert vorliegt. Bevorzugt werden hier die üblichen Kondensationsmittel wie Carbodiimide z.B. N,N′-Diethyl-, N,N′-Dipropyl-, N,N′-Diisopropyl-, N,N′-Dicyclohexylcarbodiimid, N-(3-Dimethylaminoisopropyl)-N′-ethylcarbodiimid-Hydrochlorid, oder Carbonylverbindungen wie Carbonyldiimidazol, oder 1,2-Oxazoliumverbindungen wie 2-Ethyl-5-phenyl-1,2-oxazolium-3-sulfat oder 2-tert-Butyl-5-methyl-isoxazolium-perchlorat, oder Acylaminoverbindungen wie 2-Ethoxy-1-ethoxycarbonyl-1,2-dihydrochinolin, oder Propanphosphonsäureanhydrid, oder Isobutylchloroformat, oder Benzotriazolyloxy-tris(dimethylamino)phosphonium-hexafluorophosphat, und als Basen Alkalicarbonate z.B. Natrium- oder Kaliumcarbonat oder -hydrogencarbonat, oder organische Basen wie Trialkylamine z.B. Triethylamin, N-Ethylmorpholin oder N-Methylpiperidin eingesetzt.

Die Verbindungen der allgemeinen Formel (III) können durch Umsetzung eines entsprechenden Bruchstückes, bestehend aus einer oder mehreren Aminosäuregruppierungen, mit einer freien, gegebenenfalls in

aktivierter Form vorliegenden Carboxylgruppe mit einem komplementierenden Bruchstück, bestehend aus einer oder mehreren Aminosäuregruppierungen, mit einer Aminogruppe, gegebenenfalls in aktivierter Form, herstellt, und diesen Vorgang gegebenenfalls so oft mit entsprechenden Bruchstücken wiederholt, bis man die gewünschten Peptide der allgemeinen Formel (III) hergestellt hat, anschließend gegebenenfalls Schutzgruppen abspaltet oder gegen andere Schutzgruppen austauscht.

Hierbei können zusätzliche reaktive Gruppen, wie z.B. Amino- oder Hydroxygruppen, in den Seitenketten der Bruchstücke gegebenenfalls durch übliche Schutzgruppen geschützt werden.

Aktivierte Carboxylgruppen sind hierbei bevorzugt:

Carbonsäureazide (erhältlich z.B. durch Umsetzung von geschützten oder ungeschützten Carbonsäurehydraziden mit salpetriger Säure, deren Salzen oder Alkylnitriten (z.B. Isoamylnitrit),

oder ungesättigte Ester, insbesondere Vinylester, (erhältlich z.B. durch Umsetzung eines entsprechenden Esters mit Vinylacetat), Carbamoylvinylester (erhältlich z.B. durch Umsetzung einer entsprechenden Säure mit einem Isoxazoliumreagenz), Alkoxyvinylester (erhältlich z.B. durch Umsetzung der entsprechenden Säuren mit Alkoxyacetylenen, bevorzugt Ethoxyacetylen),

oder Amidinoester z.B. N,N′- bzw. N,N-disubstituierte Amidinoester (erhältlich z.B. durch Umsetzung der entsprechenden Säure mit einem N,N′-disubsituierten Carbodiimid (bevorzugt Dicyclohexylcarbodiimid, Diisopropylcarbodiimid oder N-(3-Dimethylaminopropyl)-N′-ethylcarbodiimidhydrochlorid) oder mit einem N,N-disubstituierten Cyanamid,

oder Arylester, insbesondere durch elektronenziehende Substituenten substituierte Phenylester, z.B. 4-Nitrophenyl-, 4-Methylsulfonylphenyl-, 2,4,5-Trichlorphenyl-, 2,3,4,5,6-Pentachlorphenyl-, 4-Phenyldiazophenylester (erhältlich z.B. durch Umsetzung der entsprechenden Säure mit einem entsprechend substituierten Phenol, gegebenenfalls in Anwesenheit eines Kondensationsmittels wie z.B. N,N′-Dicyclohexylcarbodiimid, Diisopropylcarbodiimid, N-(3-Dimethylaminopropyl)-N′-ethylcarbodiimid-Hydrochlorid, Isobutylchloroformat, Propanphosphonsäureanhydrid), Benzotriazolyloxytris(dimethylamino)phosphoniumhexafluorphosphat,

oder Cyanmethylester (erhältlich z.B. durch Umsetzung der entsprechenden Säure mit Chloracetonitril in Gegenwart einer Base),

oder Thioester, insbesondere Nitrophenylthioester (erhältlich z.B. durch Umsetzung der entsprechenden Säure mit Nitrothiophenolen, gegebenenfalls in Gegenwart von Kondensationsmitteln wie N,N′-Dicyclohexylcarbodiimid, Diisopropylcarbodiimid, N-(3-Dimethylaminopropyl)-N′-ethylcarbodiimid-Hydrochlorid, Isobutylchloroformat, Propanphosphonsäureanhydrid, Benzotriazolyloxytris (dimethylamino)-phosphoniumhexafluorphosphat),

oder Amino- bzw. Amidoester (erhältlich z.B. durch Umsetzung der entsprechenden Säure mit einer N-Hydroxyamino- bzw. N-Hydroxyamido-Verbindung, insbesondere N-Hydroxy-succinimid, N-Hydroxypiperidin, N-Hydroxy-phthalimid, N-Hydroxy-5-norbornen-2,3-dicarbonsäureimid oder 1-Hydroxybenzotriazol, gegebenenfalls in Anwesenheit von Kondensationsmitteln wie N,N′-Dicyclohexylcarbodiimid, Diisopropylcarbodiimid oder N-(3-Dimethylaminopropyl)- N′-ethylcarbodiimid-Hydrochlorid, Isobutylchloroformat oder n-Propanphosphonsäureanhydrid),

oder Anhydride von Säuren, bevorzugt symmetrische oder unsymmetrische Anhydride der entsprechenden Säuren, insbesondere Anhydride mit anorganischen Säuren (erhältlich z.B. durch Umsetzung der entsprechenden Säure mit Thionylchlorid, Phosphorpentoxid oder Oxalylchlorid),

oder Anhydride mit Kohlensäurehalbderivaten z.B. Kohlensäureniederalkylhalbester (erhältlich z.B. durch Umsetzung der entsprechenden Säure mit Halogenameisensäureniedrigalkylestern, z.B.Chlorameisensäuremethylester, -ethylester, -propylester, -isopropylester, -butylester oder -isobutylester oder mit 1-Niedrigalkoxycarbonyl-2-niedrigalkoxy-1,2-dihydro-chinolin, z.B. 1-Methoxycarbonyl-2-ethoxy-1,2-dihydrochinolin),

oder Anhydride mit Dihalogenphosphorsäuren (erhältlich z.B. durch Umsetzung der entsprechenden Säure mit Phosphoroxychlorid),

oder Anhydride mit Phosphorsäurederivaten oder Phosphorigsäurederivaten, (z.B. Propanphosphonsäureanhydrid, H. Wissman und H.J. Kleiner, Angew. Chem. Int. Ed. 19, 133 (1980))

oder Anhydride mit organischen Carbonsäuren (erhältlich z.B. durch Umsetzung der entsprechenden Säuren mit einem gegebenenfalls substituierten Niederalkan- oder Phenylalkancarbonsäurehalogenid, insbesondere Phenylessigsäure, Pivalinsäure- oder Trifluoressigsäurechlorid),

oder Anhydride mit organischen Sulfonsäuren (erhältlich z.B. durch Umsetzung eines Alkalisalzes einer entsprechenden Säure mit einem Sulfonsäurehalogenid, insbesondere Methan-, Ethan-, Benzol- oder Toluolsulfonsäurechlorid),

oder symmetrische Anhydride (erhältlich z.B. durch Kondensation entsprechender Säuren, gegebenenfalls in Gegenwart von Kondensationsmitteln wie N,N′-Dicyclohexylcarbodiimid, Diisopropylcarbodiimid, N-(3-Dimethylaminopropyl)-N′-ethylcarbodiimid-Hydrochlorid Isobutylchloroformat, Propanphosphonsäureanh-

ydrid oder Benzotriazolyloxy-tris(dimethylamino)phosphoniumhexafluorphosphat.

Reaktionsfähige cyclische Amide sind insbesondere Amide mit fünfgliedrigen Heterocyclen mit 2 Stickstoffatomen und gegebenenfalls aromatischem Charakter, bevorzugt Amide mit Imidazolen oder Pyrazolen (erhältlich z.B. durch Umsetzung der entsprechenden Säuren mit N,N′-Carbonyldiimidazol oder - gegebenenfalls in Gegenwart von Kondensationsmitteln wie z.B. N,N′-Dicyclohexylcarbodiimid, N,N′-Diisopropylcarbodiimid, N-(3-Dimethylaminopropyl)-N′-ethylcarbodiimid-Hydrochlorid, Isobutylchloroformat, Propanphosphonsäureanhydrid, Benzotriazolyloxy-tris(dimethylamino)phosphonium-hexafluorphosphat- mit z.B. 3,5-Dimethyl-pyrazol, 1,2,4-Triazol oder Tetrazol.

Die eingesetzten Aminosäuren in der Definition B, D, E, F und somit auch die Verbindungen der allgemeinen Formel (IV) sind an sich bekannt oder können nach bekannten Methoden erhalten werden bzw. sind natürlich vorkommende Aminosäuren (Houben-Weyls, "Methoden der organischen Chemie, " Band XV/1 und 2).

Die Reduktion kann entweder mit Katalysatoren wie Palladiumhydroxid oder Palladium/Kohlenstoff oder über eine katalytische Transferhydrierung in an sich bekannter Weise durchgeführt werden (vgl. Tetrahedron 41, 3479 (1985), 3463 (1985), Synthesis 1987, 53).

Für die Nitron-Cycloaddition eignen sich die üblichen organischen Lösemittel, die sich unter den Reaktionsbedingungen nicht verändern. Hierzu gehören Ether wie Diethylether, Dioxan, Tetrahydrofuran, Glykol-diethylether oder Kohlenwasserstoffe wie Benzol, Toluol, Xylol, Mesitylen oder Erdölfraktionen oder Essigsäure-n-butylester, Bevorzugt sind Benzol, Toluol, Xylol oder Mesitylen.

Die Reaktion kann in einem Temperaturbereich vokn 0°C bis 200°C, bevorzugt bei 30°C bis 90°C und bei erhöhtem oder normalen Druck durchgeführt werden.

Die Verbindungen der allgemeinen Formeln (V) und (VI) sind an sich bekannt oder können nach üblicher Methode hergestellt werden.

(Chem. Pharm. Bull. 30, 1921 (1982), J.J. Tufariello in 1,3-Dipolar-Cycloaddition Chemistry, Vol. 2, id.A. Padwa p.83-168, John Wiley (1984), R. Huisgen, H. Seidel, J. Brüning, Chem. Ber. 102, 1102 (1969)).

Die erfindungsgemäßen Verbindungen besitzen eine kreislaufbeeinflussende Wirkung. Sie können deshalb in Arzneimitteln zur Behandlung des Bluthochdrucks und der Herzinsuffizienz eingesetzt werden.

In vitro Test

Die inhibitorische Stärke der erfindungsgemäßen Peptide gegen endogenes Renin vom Humanplasma wird in vitro bestimmt. Gepooltes Humanplasma wird unter Zusatz von Ethylendiamintetraessigsäure (EDTA) als Antikoagulanz erhalten und bei -20°C gelagert. Die Plasmareninaktivität (PRA) werden als Bildungsrate von Angiotensin I aus endogenem Angiotensinogen und Renin nach Inkubation bei 37°C bestimmt. Die Reaktionslösung enthält 150 µl Plasma, 3 µl 6,6%ige 8-Hydroxychinolinsulfatlösung, 3 µl 10%ige Dimercaprollösung und 144 µl Natriumphosphatpuffer (0,2 M; 0,1% EDTA; pH 5,6) mit oder ohne den erfindungsgemäßen Stoffen in verschiedenen Konzentrationen. Das pro Zeiteinheit gebildete Angiotensin I wird mit einem Radioimmunoassay (Sorin Biomedica, Italien) bestimmt. Die prozentuale Inhibition der Plasmareninaktivität wird berechnet durch Vergleich der hier beanspruchten Substanzen. Der Konzentrationsbereich, in dem die hier beanspruchten Substanzen eine 50% Inhibition der Plasmareninaktivität zeigen, liegen zwischen $10^{-4}$ bis $10^{-9}$ M.

Anwendungsbeispiele

| Beispiel Nr. | % Inhib. | $IC_{50}$(M) |
|---|---|---|
| 31 | 31 | |
| 35 | 34 | |
| 39 | 67 | |
| 77 | 96 | $1,1 \times 10^{-5}$ |
| 79 | 95 | $2,5 \times 10^{-6}$ |
| 82 | 89 | $1,4 \times 10^{-6}$ |
| 84 | 100 | $9,2 \times 10^{-8}$ |
| 85 | 100 | $2,4 \times 10^{-7}$ |
| 86 | 92 | $9,0 \times 10^{-6}$ |
| 94 | 100 | $3,7 \times 10^{-6}$ |
| 96 | 100 | $1,0 \times 10^{-6}$ |
| 97 | 100 | $2,2 \times 10^{-8}$ |

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel, Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgier mitteln und/oder Dispergiermitteln, wobei z.B. im Fall der Benutzung von Waser als Verdünnungsmittel gegebenenfalls organische Lösungsmittel als Hilfslösungsmittel verwendet werden können.

Als Hilfsstoffe seien beispielsweise aufgeführt:
Wasser, nicht-toxische organische Lösungsmittel, wie Paraffine (z.B. Erdölfraktionen), pflanzliche Öle (z.B. Erdnuß/Sesamöl), Alkohole (z.B: Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art der Applikation, aber auch aufgrund

der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

Folgende Laufmittel-Systeme wurden benutzt:

(a) 10:1 Methylenchlorid/Methanol
(b) 20:1 Methylenchlorid/Methanol
(c) 40:1 Methylenchlorid/Methanol
(d) 2:1 n-Hexan/Ether
(e) 9:1:0,1 Methylenchlorid/Methanol/Ameisensäure
(f) 20:1 Chloroform/Aceton
(g) 15:1 Methylenchlorid/Methanol
(h) 20:1 Chloroform/Methanol
(i) 9:1:0,1 Methylenchlorid/Methanol/konz. Ammoniak
(j) 1:1 n-Hexan/Essigester
(k) 30:1 Methylenchlorid/Methanol

| Abkürzungen | |
|---|---|
| Leu | Leucin |
| Boc | tert.-Butyloxycarbonyl |
| Ile | Isoleucin |
| NEM | N-Ethylmorpholin |
| HOBT | 1-Hydroxy-1H-benzotriazol |
| DCC | Dicyclohexylcarbodiimid |
| AMP | 2-Aminomethylpyridin |
| Phe | Phenylalanin |
| His | Histidin |
| Pro | Prolin |
| CHxAla | 3-Cyclohexylalanin |

Die folgenden Ausführungsbeispiele der Tabellen 1 bis 3 (Ex 1 bis 76) zeigen Vorstufen und Zwischenprodukte und die Ausführungsbeispiele der Tabellen 4 bis 6 (Ex 77 bis 109) beschreiben Verbindungen der Formel (I).

Beispiel 1

L-Phenylalaninmethylester-Hydrochlorid

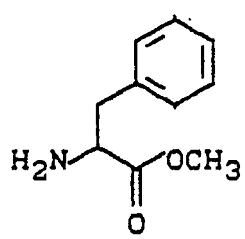

x HCl

99 g (0,83 mol) Thionylchlorid wurden bei -10° C zu 600 ml absolutem Methanol getropft. Anschließend wurden 100 g (0.605 mol) L-Phenylalanin eingetragen und das Reaktionsgemisch zwei Stunden lang unter

Rückfluß erhitzt. Die Lösung wurde eingeengt, der Rückstand in einer gerade ausreichenden Menge absolutem Methanol gelöst und das Produkt durch Zusatz von absolutem Ether ausgefällt.
Ausbeute: 119 g = 91% d.Th.
Fp. 159° C.

Beispiel 2

L-Leucinmethylester-Hydrochlorid

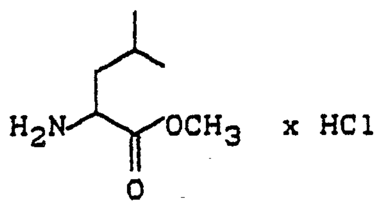

Beispiel 2 wird analog der Vorschrift von Beispiel 1 hergestellt.
Ausbeute: 118,4 g = 86% d. Th. Fp. 148° C

Beispiel 3

tert.-Butoxycarbonyl-L-Phenylalaninmethylester

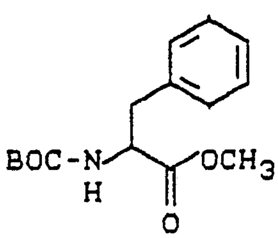

119 g (0.553 mol) der Verbindung aus Beispiel 1 in 750 ml DMF wurden bei 5° C nacheinander mit 116 ml (0.834 mol) Triethylamin und 146 g (0.669 mol) Di-tert.-butylpyrocarbonat versetzt und das Gemisch über Nacht bei Raumtemperatur gerührt. Das Gemisch wurde filtriert, das Filtrat eingeengt, der Rückstand in 1:1 Wasser/Essigester aufgenommen und mit verdünnter Salzsäure auf pH 3 gestellt. Die wäßrige Phase wurde dreimal mit Essigester extrahiert, die vereinigten org. Phasen zweimal mit ges. Kochsalzlösung gewaschen, über Natriumsulfat getrocknet und eingeengt.
Ausbeute: 154 g = 100% d. Th.
$R_F$ (i): 0,86

Beispiel 4

tert.-Butoxycarbonyl-L-Leucinmethylester

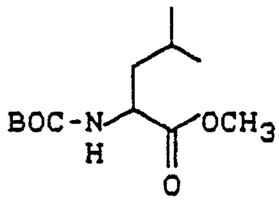

Beispiel 4 wird analog der Vorschrift von Beispiel 3 hergestellt.
Ausbeute: 69 g = 100% d. Th.
$R_F$ (a): 0,90

Beispiel 5

tert.-Butoxycarbonyl-L-Phenylalaninol

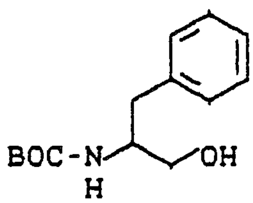

Zu einer Suspension von 31.32 g (0.828 mol) Natriumborhydrid in 200 ml abs. Tetrahydrofuran wurden in Portionen 110.8 g (0.828 mol) Lithiumiodid gegeben. Anschließend wurde eine Lösung von 190.2 g (0.552 mol) der Verbindung aus Beispiel 3 in 320 ml abs. Tetrahydrofuran zugetropft und die Suspension bei 40 °C über Nacht gerührt. Das Gemisch wurde vorsichtig in 10%-ige Zitronensäurelösung gegossen, viermal mit Essigester extrahiert, die vereinigten org. Phasen zweimal mit ges. Kochsalzlösung gewaschen, über Natriumsulfat getrocknet und eingeengt. Das Rohprodukt wurde aus Essigester/n-Hexan umkristallisiert.
Ausbeute: 118 g = 85% d. Th.
Fp. 95 °C.
$R_F$ (a): 0,61

Beispiel 6

tert.-Butoxycarbonyl-L-3-cyclohexylalaninol

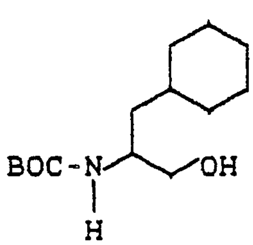

Beispiel 6 wird analog der Vorschriften von Beispiel 5 hergestellt.
Ausbeute: 123,7 g = 89% d. Th.
$R_F$ (b): 0,44

Beispiel 7

tert.-Butoxycarbonyl-L-leucinol

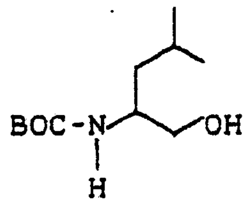

Beispiel 7 wird analog der Vorschrift von Beispiel 5 hergestellt.
Ausbeute: 33,2 g = 87,5% d. Th.
$R_F$ (b): 0,37

Beispiel 8

tert.-Butoxycarbonyl-L-phenylalaninal

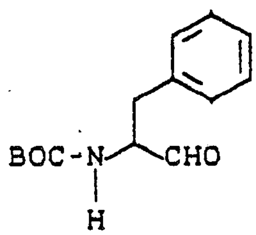

57.9 g (0.23 mol) Boc-Phenylalaninol (Beispiel 5) in 700 ml DMSO wurden unter Eiskühlung mit 96 ml (0.69 mol) Triethylamin und 114 g (0.69 mol) Pyridin-$SO_3$-Komplex versetzt und das Gemisch eine Stunde lang bei Raumtemperatur gerührt. Die Lösung wurde auf Eis gegossen, viermal mit Ether extrahiert, die vereinigten org. Phasen zweimal mit 10%-iger Zitronensäure-, zweimal mit 5%-iger Natriumhydrogencarbonat- und zweimal mit halbgesättigter Kochsalz-Lösung gewaschen, über Natriumsulfat getrocknet und eingeengt. Das Rohprodukt wurde direkt weiterverarbeitet.
Ausbeute: 52.6 g = 91.9 % d.Th.
$R_F$ (a): 0,71

Beispiel 9

tert.-Butoxycarbonyl-L-3-cyclohexylalaninal

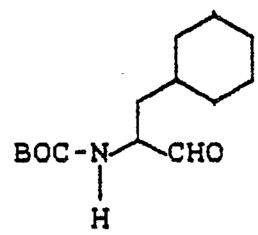

Beispiel 9 wird analog der Vorschrift von Beispiel 8 hergestellt.
Ausbeute: 65 g = 95% d.Th.
$R_F$ (b): 0,57

Beispiel 10

tert.-Butoxycarbonyl-L-leucinal

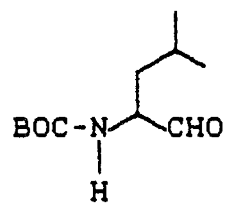

Beispiel 10 wird analog der Vorschrift von Beispiel 8 hergestellt.
Ausbeute: 56 g = 94% d.Th.
$R_F$ (a): 0,71

Beispiel 11

4-(S)-Boc-amino-5-phenyl-2-pentensäureethylester

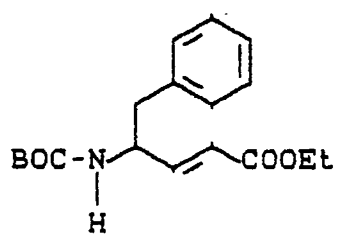

Zu einer Lösung von 32.8 g (0.147 mol) Triethylphosphonoacetat in 220 ml absolutem 1,2-Dimethoxyethan wurden 6.2 g (0.147 mol) Lithiumchlorid gegeben und anschließend bei 0° C langsam 18.0 g (0.140 mol) Diisopropylethylamin zugetropft. Nach 15 minütigem Rühren bei 0° C wurde eine Lösung von 36.48 g (0.139 mol) Boc-Phenylalaninal (Beispiel 8) in 200 ml abs. 1,2-Dimethoxyethan zugegeben und das Gemisch zwei Tage lang bei Raumtemperatur gerührt. Die resultierende Suspension wurde in eiskalte 10%-ige Zitronensäurelösung eingerührt, viermal mit Ether extrahiert, die vereinigten org. Phasen mit ges. Kochsalzlösung gewaschen, über Natriumsulfat getrocknet, eingeengt und das Rohprodukt über Kieselgel in 30:1 Methylenchlorid/Methanol säulenfiltriert.
Ausbeute: 36.3 g = 77.6% d.Th.

$R_F$ (k): 0,67

Beispiel 12

4-(S)-Boc-amino-5-cyclohexyl-2-pentensäureethylester

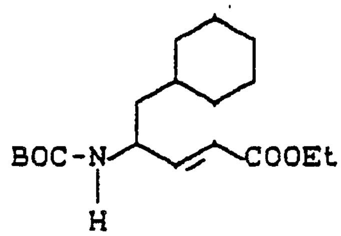

Beispiel 12 wird analog der Vorschrift von Beispiel 11 hergestellt.
Ausbeute: 14,9 g = 70,6% d.Th., Fp.: 49-50 °C.
$R_F$ (d): 0,39

Beispiel 13

4-(S)-Boc-amino-6-methyl-2-heptensäureethylester

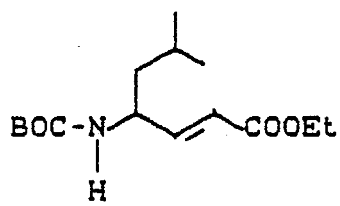

Beispiel 13 wird analog der Vorschrift von Beispiel 11 hergestellt.
Ausbeute: 23 g = 72,9% d.Th. Wachs.
$R_F$ (c): 0,58

Beispiel 14

tert.-Butoxycarbonyl-L-Phenylalanin

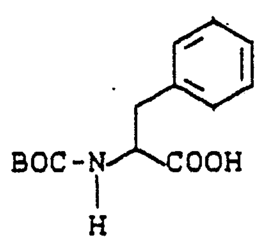

50 g (0.30 mol) L-Phenylalanin in 900 ml 2:1 Dioxan/Wasser wurden mit 151 ml 2 N Natronlauge und

88.7 g (0.366 mol) 90%-igem Di-tert.-butylpyrocarbonat versetzt. Das Gemisch wurde über Nacht bei Raumtemperatur gerührt, dann auf ca. ein Drittel des ursprünglichen Volumens eingeengt, der Rückstand mit Wasser verdünnt und bie pH 9 zweimal mit Ether extrahiert. Die wäßrige Phase wurde mit verdünnter Salzsäure auf pH 3 angesäuert, sechsmal mit Essigester extrahiert, die vereinigten Essigester-Phasen mit ges. Kochsalzlösung gewaschen, über Natriumsulfat getrocknet und eingeengt. Der Rückstand wurde mit n-Hexan bis zur Kristallisation verrieben und das Produkt abgesaugt.
Ausbeute: 77 g = 95.5% d.Th.
Fp. 84° C.

Beispiel 15

tert.-Butoxycarbonyl-L-3-cyclohexylalanin

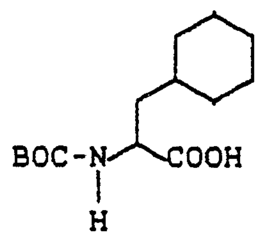

299 g (1.126 mol) der Verbindung aus Beispiel 14 in 1.1 l Methanol wurden unter Zusatz von 30 ml Eisessig bei 30-40° C mit 30 g 5 %igem RH/C-Katalysator und einem Wasserstoffdruck von 40-50 bar hydriert. Nach beendeter Reaktion wurde der Katalysator abfiltriert und die Lösung eingeengt.
Ausbeute: 306 g = 100 % d.Th.

Beispiel 16

tert.-Butoxycarbonyl-L-3-cyclohexylalaninmethylester

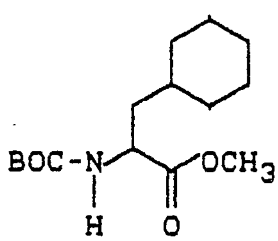

242.7 g (0.856 mol) der Verbindung aus Beispiel 15 in 1.2 l DMF wurden mit 177.4 g (1.283 mol) Kaliumcarbonat und 56 ml (0.898 mol) Methyliodid versetzt. Nach zugabe von weiteren 500 ml DMF wurde das Gemisch über Nacht bei Raumtemperatur kräftig gerührt, dann filtriert, das Filtrat eingeengt, der Rückstand in Wasser aufgenommen und viermal mit Ether extrahiert. Die vereinigten organischen Phasen wurden mit ges. Kochsalzlösung gewaschen, über Natriumsulfat getrocknet und eingeengt.
Ausbeute: 232 g = 95 % d.Th.
$R_F$ (b): 0,82

Beispiel 17

tert.-Butoxycarbonyl-L-isoleucin

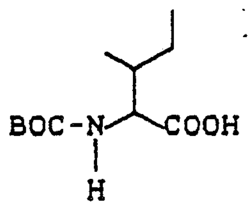

Beispiel 17 wird analog der Vorschrift von Beispiel 14 hergestellt.
Ausbeute: 168,4 g = 95,5% d.Th.
Fp. 70 °C

Beispiel 18

N-Methylenbenzylamin-N-oxid

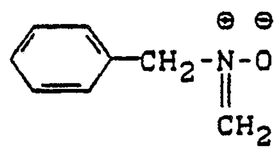

65 g (0.313 mol) N-Benzylhydroxylamin in 630 ml Ether wurden unter intensivem Rühren mit 125 ml (1.585 mol) 35%-iger wäßriger Formaldehydlösung versetzt. Es wurde noch eine Stunde lang nachgerührt, dann die organische Phase abgetrennt, über Natriumsulfat getrocknet und eingeengt. Das Rohprodukt wurde direkt weiterverarbeitet.
Ausbeute: 65.7 g = 92% d.Th.

Beispiel 19

2-Benzyl-5-(1-(S)-Boc-amino-2-phenylethyl)-isoxazolidin-4-carbonsäureethylester

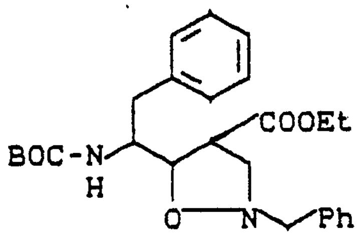

20 g (0.063 mol) der Verbindung aus Beispiel 11 und 16.9 g (0.125 mol) der Verbindung aus Beispiel 18 in 300 ml Toluol wurden sieben Stunden lang bei 60 °C und anschließend über Nacht bei Raumtemperatur gerührt. Nach Zusatz von weiteren 4.16 g (0.031 mol) der Verbindung aus Beispiel 18 in 25 ml Toluol wurde nochmals zwei Stunden lang auf 60 °C erhitzt, dann das Reaktionsgemisch zweimal mit halbgesättigter Kochsalzlösung gewaschen, über Natriumsulfat getrocknet und eingeengt. Säulenchromatographie des Rohprodukts an Kieselgel in 2:1 Hexan/Ether ergab 12.65 g (44.5% d.Th.) des unpolaren und 4.26 g (15.0% d.Th.) des polaren Isomeren.

Gesamtausbeute: 16.91 g = 59.5% d.Th.
$R_F$ (d): 0,25 bzw. 0,17

Beispiel 20

2-Benzyl-5-(1-(S)-Boc-amino-2-cyclohexylethyl)-isoxazolidin-4-carbonsäureethylester

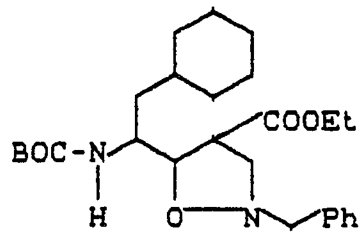

Beispiel 20 wird analog der Vorschrift von Beispiel 19 hergestellt.
Ausbeute: 16,12 g = 57% d.Th.
$R_F$ (d): 0,32 bzw. 0,25

Beispiel 21

2-Benzyl-5-(1-(S)-Boc-amino-3-methylbutyl)-isoxazolidin-4-carbonsäureethylester

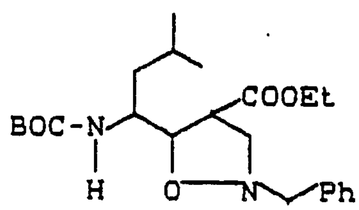

Beispiel 21 wird analog der Vorschrift von Beispiel 19 hergestellt.
Ausbeute: 18,55 g = 63,1% d.Th.
$R_F$ (d): 0,33 bzw. 0,25

Beispiel 22

2-Benzyl-5-(1-(S)-Boc-amino-3-methylbutyl)-isoxazolidin-4-carbonsäure

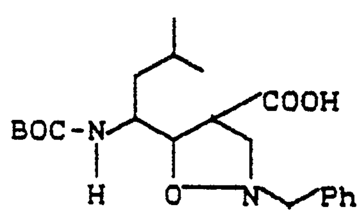

5.4 g (12.8 mmol) der Verbindung aus Beispiel 21 (Isomerengemisch) in 60 ml 2:1 Dioxan/Wasser wurden drei Stunden lang mit 14.1 ml 1 N Natronlauge bei Raumtemperatur gerührt und anschließend über Nacht im Kühlschrank stehengelassen. Die Lösung wurde auf ein Drittel des ursprünglichen Volumens eingeengt, mit Wasser verdünnt und bie pH 12 zweimal mit Ether extrahiert. Die wäßrige Phase wurde mit verdünnter Salzsäure auf pH 5 gebracht, viermal mit Essigester extrahiert, die vereinigten Essigester-Phasen mit ges. Kochsalzlösung gewaschen, über Natriumsulfat getrocknet und eingeengt.
Ausbeute: 4.75 g = 94.4% d.Th.
$R_F$ (e): 0,45

Beispiel 23

2-Benzyl-5-(1-(S)-Boc-amino-2-phenylethyl)-isoxazolidin-4-carbonsäure

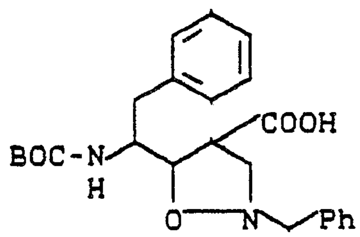

Beispiel 23 wird analog der Vorschrift von Beispiel 22 hergestellt.
Ausbeute: 12,74 g = 92,1% d.Th.
$R_F$ (e): 0,46

Beispiel 24

2-Benzyl-5-(1-(S)-Boc-amino-2-cyclohexylethyl)-isoxazolidin-4-carbonsäure

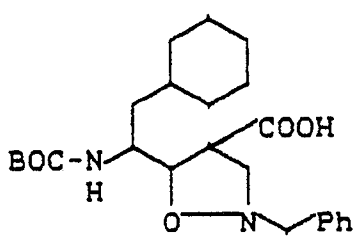

Beispiel 24 wird analog der Vorschrift von Beispiel 22 hergestellt.
Ausbeute: 3,9 g = 94% d.Th.
$R_F$ (e): 0,40 bzw. 0,31

Beispiel 25

Boc-L-isoleucin-2-aminomethylpyridylamid

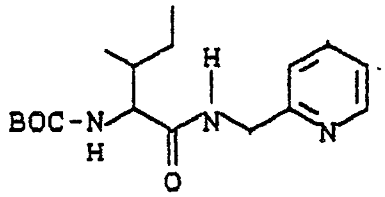

30 g (0.13 mol) der Verbindung aus Beispiel 17 in 250 ml Methylenchlorid wurden bei 0° C nacheinander mit 14.75 g (0.136 mol) 2-Picolylamin, 123 ml (0.886 mol) Triethylamin und 116 ml einer 50%-igen Lösung von Propan phosphonsäureanhydrid in Methylenchlorid versetzt. Die Lösung wurde über Nacht bei Raumtemperatur gerührt, dann in eiskalte Natriumhydrogencarbonat-Lösung gegossen, die org. Phase noch einmal mit 5%-iger Natriumhydrogencarbonat- und einmal mit ges. Kochsalz-Lösung gewaschen, über Natriumsulfat getrocknet, eingeengt und das Rohprodukt über Kieselgel in 15:1 Methylenchlorid/Methanol säulenfiltriert.
Ausbeute: 35,33 g = 84.6% d.Th.
$R_F$ (g): 0,52

Beispiel 26

L-Isoleucin-2-aminomethylpyridylamid-Dihydrochlorid

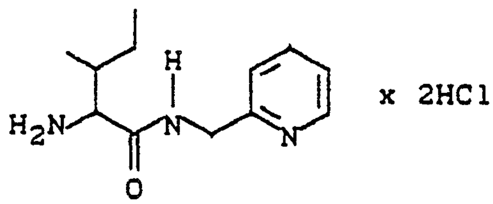

35.33 g (0.11 mol) der Verbindung aus Beispiel 25 wurden mit 150 ml 4 N HCl/Dioxan und 40 ml absolutem Methanol 75 Minuten lang bei 0° C und drei Stunden bei Raumtemperatur gerührt. Die Lösung wurde eingeengt, der Rückstand mehrmals mit absolutem Ether versetzt, jeweils wieder eingeengt und das Produkt im Hochvakuum getrocknet.
Ausbeute: 33 g = 100% d.Th.

Beispiel 27

N-[2-Benzyl-5-(1-(S)-Boc-amino-2-phenylethyl)-isoxazolidin-4-carbonyl]-L-isoleucin-methylester

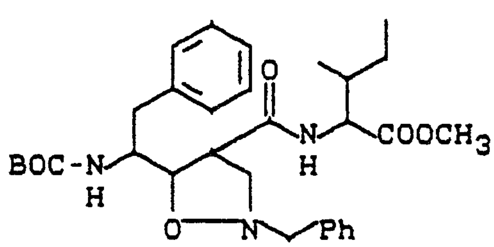

12.7 g (29.77 mmol) der Verbindung aus Beispiel 23 (Isomerengemisch) in 100 ml Methylenchlorid wurden bei 0° C nacheinander mit 5.95 g (32.75 mmol) L-Ile-$OCH_3$ x HCl, 3.45 g (34.24 mmol) Triethylamin, 5.47 g (35.73 mmol) HOBt und 7.06 g (34.24 mmol) DCC versetzt und das Reaktionsgemisch über Nacht

bei Raumtemperatur gerührt. Der entstandene Niederschlag wurde abfiltriert, das Filtrat zweimal mit 5%-iger Natriumhydrogencarbonat- und einmal mit ges. Kochsalz-Lösung gewaschen, über Natriumsulfat getrocknet und eingeengt. Das Rohprodukt wurde durch Säulenchromatographie an Kieselgel in 40:1 Methylenchlorid/Methanol gereinigt.
Ausbeute: 14 g = 85 % d.Th.
$R_F$ (c) : 0,39 bzw. 0,23

Die in Tabelle 1 aufgeführten Beispiele wurden analog der Vorschrift von Beispiel 27 dargestellt.

Tabelle 1

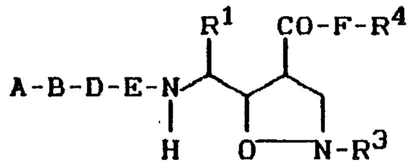

| Beispiel Nr. | A | B | D | E | F | $R^1$ | $R^3$ | $R^4$ | $R_F$ |
|---|---|---|---|---|---|---|---|---|---|
| 28 | H | - | - | - | Ile | $CH_2-C_6H_5$ | $CH_2-C_6H_5$ | $OCH_3$ | 0,71 (i) |
| 29 | BOC | - | - | - | Ile | $CH_2-C_6H_5$ | $CH_2-C_6H_5$ | $-NH-CH_2$ 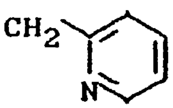 | 0,39 (h) |
| 30 | H | - | - | - | Ile | $CH_2-C_6H_5$ | $CH_2-C_6H_5$ | $-NH-CH_2$ 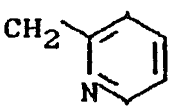 | 0,55 (i) |
| 31 | BOC | - | Phe | His | Ile | $CH_2-C_6H_5$ | $CH_2-C_6H_5$ | $OCH_3$ | 0,53 (a) |
| 32 | BOC | Pro | Phe | His | Ile | $CH_2-C_6H_5$ | $CH_2-C_6H_5$ | $OCH_3$ | 0,46 (a) |
| 33 | BOC | - | Phe | His | Ile | $CH_2-C_6H_5$ | $CH_2-C_6H_5$ | $-NH-CH_2$ 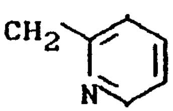 | 0,33 (i) |
| 34 | BOC | Pro | Phe | His | Ile | $CH_2-C_6H_5$ | $CH_2-C_6H_5$ | $-NH-CH_2$ 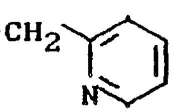 | 0,38 (i) |

Beispiel 35:

N-[2-Benzyl-5-(1-(S)-Boc-amino-2-cyclohexylethyl)-isoxazolidin-4-carbonyl]-L-isoleucin-methylester

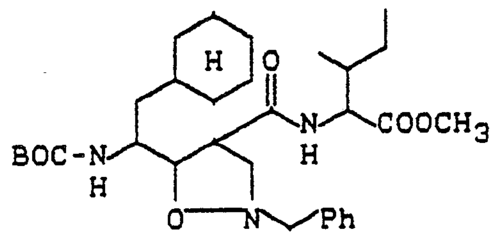

3.0 g (6.94 mmol) der Verbindung aus Beispiel 24 (Isomerengemisch) in 35 ml Methylenchlorid wurden bei 0 °C nacheinander mit 1.38 g (7.63 mmol) L-Ile-$OCH_3$ x HCl, 0.92 g (7.98 mmol) N-Ethylmorpholin, 1.27 g (8.32 mmol) HOBt und 1.72 g (8.32 mmol) DCC versetzt und das Reaktionsgemisch über Nacht bei Raumtemperatur gerührt. Der entstandene Niederschlag wurde abfiltriert, das Filtrat zweimal mit 5%-iger Natriumhydrogencarbon- und einmal mit ges. Kochsalz-Lösung gewaschen, über Natriumsulfat getrocknet und eingeengt. Das Rohprodukt wurde durch Säulenchromatographie an Kieselgel in 1:1 n-Hexan/Essigester gereinigt.

Ausbeute: 3.5 g = 90% d.Th.

$R_F$ (j): 0,60 bzw. 0,49

Die in Tabelle 2 aufgeführten Beispiele wurden analog der Vorschrift von Beispiel 35 hergestellt.

Tabelle 2

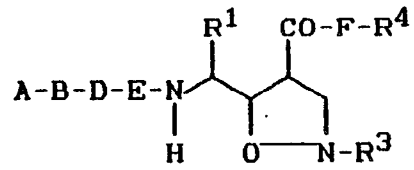

| Beispiel Nr. | A | B | D | E | F | $R^1$ | $R^3$ | $R^4$ | $R_F$ |
|---|---|---|---|---|---|---|---|---|---|
| 36 | H | - | - | - | Ile | $CH_2-C_6H_{11}$ | $CH_2-C_6H_5$ | $OCH_3$ | |
| 37 | BOC | - | - | - | Ile | $CH_2-C_6H_{11}$ | $CH_2-C_6H_5$ | 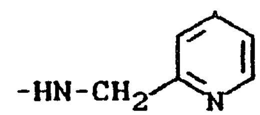 | 0,44 bzw. 0,31 (h) |
| 38 | H | - | - | - | Ile | $CH_2-C_6H_5$ | $CH_2-C_6H_5$ | 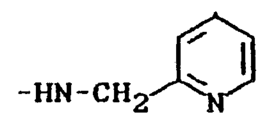 | 0,50 (i) |
| 39 | BOC | - | Phe | His | Ile | $CH_2-C_6H_{11}$ | $CH_2-C_6H_5$ | $OCH_3$ | 0,45 (a) |
| 40 | H | - | Phe | His | Ile | $CH_2-C_6H_{11}$ | $CH_2-C_6H_5$ | $OCH_3$ | |
| 41 | BOC | Pro | Phe | His | Ile | $CH_2-C_6H_{11}$ | $CH_2-C_6H_5$ | $OCH_3$ | 0,55 (i) |
| 42 | BOC | - | Phe | His | Ile | $CH_2-C_6H_{11}$ | $CH_2-C_6H_5$ | 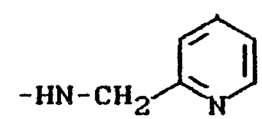 | 0,54 (i) |

<u>Tabelle 2</u> (Fortsetzung)

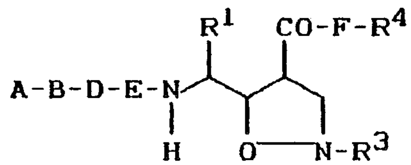

| Beispiel Nr. | A | B | D | E | F | $R^1$ | $R^3$ | $R^4$ | $R_F$ |
|---|---|---|---|---|---|---|---|---|---|
| 43 | BOC | Pro | Phe | His | Ile | $CH_2-C_6H_{11}$ | $CH_2-C_6H_5$ | 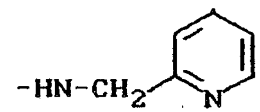 | 0,38 (i) |
| 44 | BOC | - | - | - | - | $CH_2-C_6H_{11}$ | $CH_2-C_6H_5$ | $NH-iC_4H_9$ | 0,39 bzw. 0,31 (c) |
| 45 | H | - | - | - | - | $CH_2-C_6H_{11}$ | $CH_2-C_6H_5$ | $NH-iC_4H_9$ | |
| 46 | BOC | - | Phe | His | - | $CH_2-C_6H_{11}$ | $CH_2-C_6H_5$ | $NH-iC_4H_9$ | 0,48 (i) |
| 47 | BOC | - | - | - | - | $CH_2-C_6H_{11}$ | $CH_2-C_6H_5$ | $NH-C_2H_4-C_6H_5$ | 0,43 bzw. 0,34 (c) |
| 48 | H | - | - | - | - | $CH_2-C_6H_{11}$ | $CH_2-C_6H_5$ | $NH-C_2H_4-C_6H_5$ | |
| 49 | BOC | - | Phe | His | - | $CH_2-C_6H_{11}$ | $CH_2-C_6H_5$ | $NH-C_2H_4-C_6H_5$ | 0,47 (i) |
| 50 | - | - | 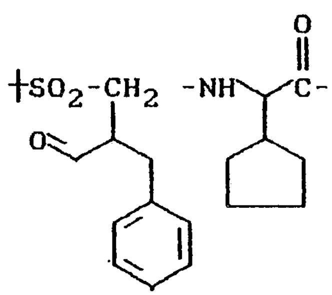 | | Ile | $CH_2-C_6H_{11}$ | $CH_2-C_6H_5$ | 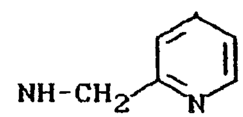 | 0,36 (b) |

Tabelle 2 (Fortsetzung)

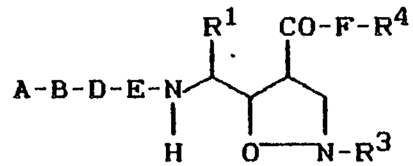

| Beispiel Nr. | A | B | D | E | F | $R^1$ | $R^3$ | $R^4$ | $R_F$ |
|---|---|---|---|---|---|---|---|---|---|
| 51 | BOC | - | Phe | 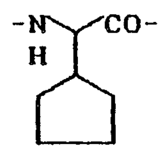 | Ile | $CH_2-C_6H_{11}$ | $CH_2-C_6H_5$ | 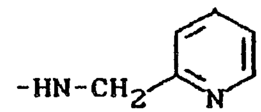 | 0,33 (b) |
| 52 | BOC | - | 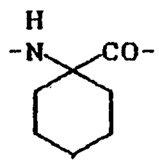 | His | Ile | $CH_2-C_6H_{11}$ | $CH_2-C_6H_5$ | 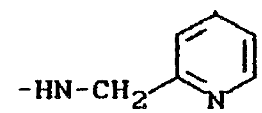 | 0,42 (i) |
| 53 | - | - | 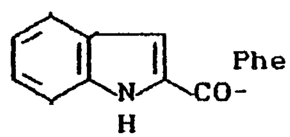 | Phe | Ile | $CH_2-C_6H_{11}$ | $CH_2-C_6H_5$ | 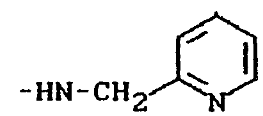 | 0,38 (b) |
| 54 | - | - | 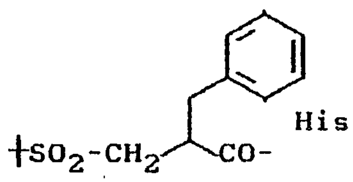 | His | Ile | $CH_2-C_6H_{11}$ | $CH_2-C_6H_5$ | 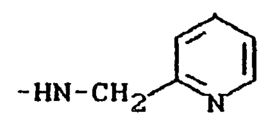 | 0,53 bzw. 0,51 (i) |

Tabelle 2 (Fortsetzung)

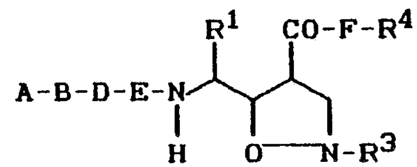

| Beispiel Nr. | A | B | D | E | F | $R^1$ | $R^3$ | $R^4$ | $R_F$ |
|---|---|---|---|---|---|---|---|---|---|
| 55 | Boc | - | 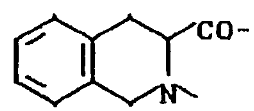 | His | Ile | $CH_2-C_6H_{11}$ | $CH_2-C_6H_5$ | 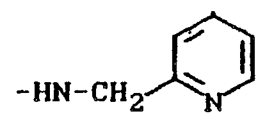 | 0,50 (i) |

Beispiel 56

N-[2-Benzyl-5-(1-(S)-Boc-amino-3-methylbutyl)-isoxazolidin-4-carbonyl]-L-isoleucin-methylester

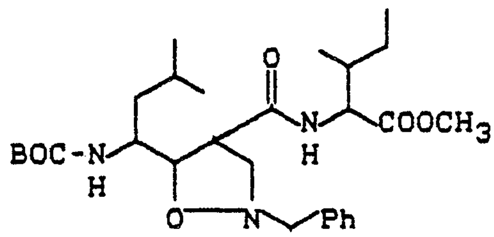

2.75 g (7.01 mmol) der Verbindung aus Beispiel 22 (Isomerengemisch) in 30 ml Methylenchlorid wurden bei 0 °C nacheinander mit 1.4 g (7.71 mmol) L-Ile-$OCH_3$ x HCl, 0.928 g (8.06 mmol) N-Ethylmorpholin, 1.29 g (8.41 mmol) HOBt und 1.73 g (8.41 mmol) DCC versetzt und das Reaktionsgemisch über Nacht bei Raumtemperatur gerührt. Der entstandene Niederschlag wurde abfiltriert, das Filtrat zweimal mit 5%-iger Natriumhydrogencarbonat- und einmal mit ges. Kochsalz-Lösung gewaschen, über Natriumsulfat getrocknet und eingeengt. Säulenchromatographie des Rohproduktes an Kieselgel in 20:1 Chloroform/Aceton ergab 1.83 g (50.3% d.Th.) des unpolaren und 1.11 g (30.5% d.Th.) des polaren Isomeren.

Gesamtausbeute: 2.94 g = 80.8% d.Th.

$R_F$ (f): 0,42 bzw. 0,25

Die in Tabelle 3 aufgeführten Beispiel wurden analog der Vorschrift von Beispiel 56 hergestellt.

<u>Tabelle 3</u>

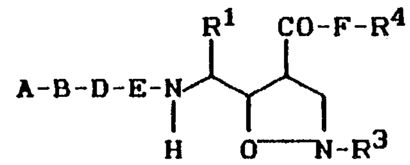

| Beispiel Nr. | A | B | D | E | F | $R^1$ | $R^3$ | $R^4$ | $R_F$ |
|---|---|---|---|---|---|---|---|---|---|
| 57 | H | - | - | - | Ile | $iC_4H_9$ | $CH_2-C_6H_5$ | $OCH_3$ | 0,47 (a) |
| 58 | BOC | - | - | - | Ile | $iC_4H_9$ | $CH_2-C_6H_5$ | 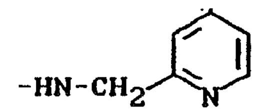 | 0,47 bzw. 0,37 (h) |
| 59 | H | - | - | - | Ile | $iC_4H_9$ | $CH_2-C_6H_5$ | 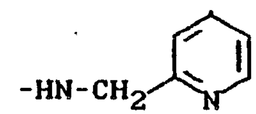 | 0,31 (a) |
| 60 | BOC | - | Phe | His | Ile | $iC_4H_9$ | $CH_2-C_6H_5$ | $OCH_3$ | 0,46 (a) |
| 61 | H | - | Phe | His | Ile | $iC_4H_9$ | $CH_2-C_6H_5$ | $OCH_3$ | 0,23 (i) |
| 62 | BOC | Pro | Phe | His | Ile | $iC_4H_9$ | $CH_2-C_6H_5$ | $OCH_3$ | 0,45 (i) |
| 63 | BOC | - | Phe | His | Ile | $iC_4H_9$ | $CH_2-C_6H_5$ | 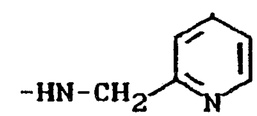 | 0,43 bzw. 0,32 (a) |
| 64 | BOC | Pro | Phe | His | Ile | $iC_4H_9$ | $CH_2-C_6H_5$ | 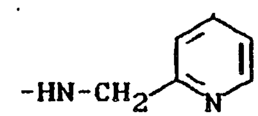 | 0,26 bzw. 0,17 (a) |

<u>Tabelle 3</u> (Fortsetzung)

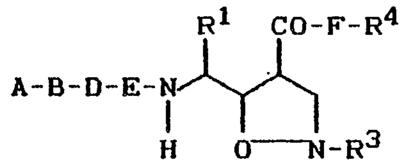

| Beispiel Nr. | A | B | D | E | F | $R^1$ | $R^3$ | $R^4$ | $R_F$ |
|---|---|---|---|---|---|---|---|---|---|
| 65 | Boc | - | - | 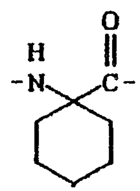 | Ile | $iC_4H_9$ | $CH_2-C_6H_5$ | 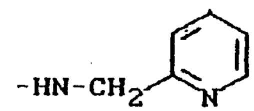 | 0,34 (b) |
| 66 | 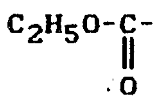 | - | Phe | His | Ile | $iC_4H_9$ | $CH_2-C_6H_5$ | 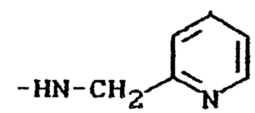 | 0,38 (i) |
| 67 | 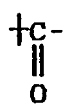 | - | Phe | His | Ile | $iC_4H_9$ | $CH_2-C_6H_5$ | 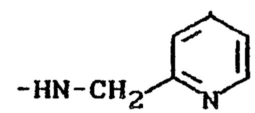 | 0,33 (i) |
| 68 | Boc | - | 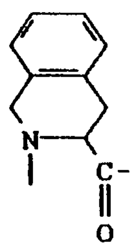 | 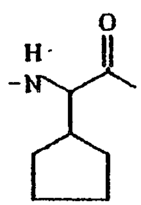 | Ile | $iC_4H_9$ | $CH_2-C_6H_5$ | 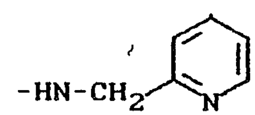 | 0,33 (b) |

Tabelle 3 (Fortsetzung)

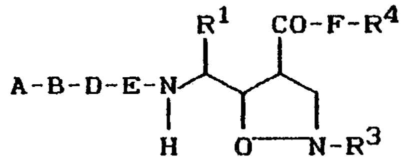

| Beispiel Nr. | A | B | D | E | F | $R^1$ | $R^3$ | $R^4$ | $R_F$ |
|---|---|---|---|---|---|---|---|---|---|
| 69 | - | - | 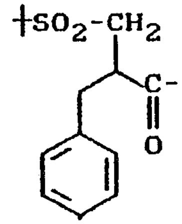 | His | Ile | $iC_4H_9$ | $CH_2-C_6H_5$ | $-HN-CH_2$ 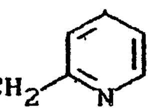 | 0,46 bzw. 0,42 (i) |
| 70 | Boc | - | 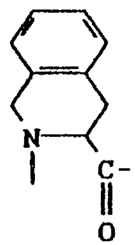 | His | Ile | $iC_4H_9$ | $CH_2-C_6H_5$ | $-HN-CH_2$ 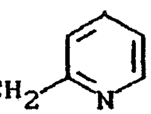 | 0,49 (i) |
| 71 | Boc | - | 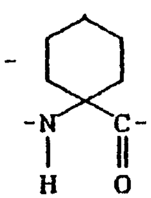 | His | Ile | $iC_4H_9$ | $CH_2-C_6H_5$ | $-HN-CH_2$ 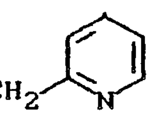 | 0,41 (i) |

<u>Tabelle 3</u> (Fortsetzung)

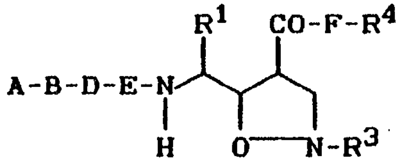

| Beispiel Nr. | A | B | D | E | F | $R^1$ | $R^3$ | $R^4$ | $R_F$ |
|---|---|---|---|---|---|---|---|---|---|
| 72 | - | - | 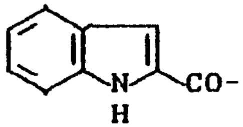 | Phe | Ile | $iC_4H_9$ | $CH_2-C_6H_5$ | 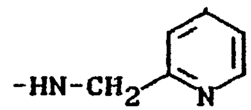 | 0,32 bzw. 0,28 (b) |
| 73 | - | - | - | 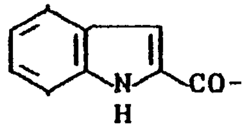 | Ile | $iC_4H_9$ | $CH_2-C_6H_5$ | 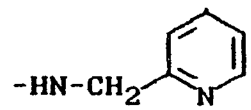 | 0,40 bzw. 0,36 (b) |
| 74 | - | - | 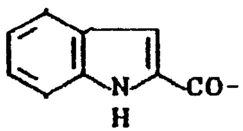 | 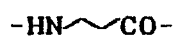 | Ile | $iC_4H_9$ | $CH_2-C_6H_5$ | 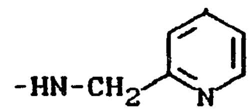 | 0,23 bzw. 0,18 (b) |
| 75 | - | - | 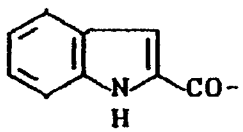 | His | Ile | $iC_4H_9$ | $CH_2-C_6H_5$ | 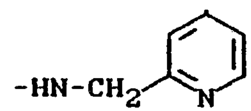 | 0,39 bzw. 0,36 (i) |
| 76 | Boc | - | Phe | 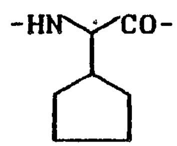 | Ile | $iC_4H_9$ | $CH_2-C_6H_5$ | 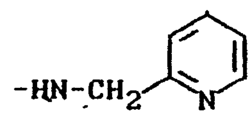 | 0,32 (b) |

Beispiel 77

N-[2-Aminomethyl-4-(S)-Boc-L-phenylalanyl-L-histidylamino-3-hydroxy-5-phenylpentanoyl]-L-isoleucinmethylester

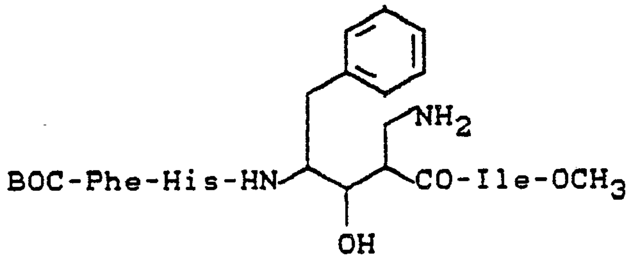

300 mg 10%-iger Pd/C-Katalysator wurden zu einer Lösung von 500 mg (0.6 mmol) der Verbindung aus Beispiel 31 und 189 mg (3 mmol) Ammoniumformiat in 25 ml Methanol gegeben und die Mischung unter Argon eine Stunde lang unter Rückfluß erhitzt. Der Katalysator wurde abfiltriert, das Filtrat eingeengt, der Rückstand in Methylenchlorid aufgenommen, einmal mit halbges. Kochsalzlösung gewaschen, über Natriumsulfat getrocknet, eingeengt, und das Rohprodukt durch Säulenchromatographie an Kieselgel in 9:1:0.1 Methylenchlorid/Methanol/konz. wäßr. Ammoniaklösung gereinigt.
Ausbeute: 380 mg = 84.7% d. Th.
$R_F$ (i): 0,17

Die in Tabelle 4 aufgeführten Beispiele wurden analog der Vorschrift von Beispiel 77 hergestellt.

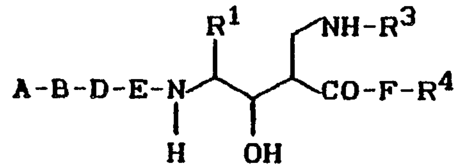

| Beispiel Nr. | A | B | D | E | F | $R^1$ | $R^3$ | $R^4$ | $R_F$ |
|---|---|---|---|---|---|---|---|---|---|
| 78 | BOC | Pro | Phe | His | Ile | $CH_2-C_6H_5$ | H | $OCH_3$ | 0,28 bzw. 0,21 (i) |
| 79 | BOC | - | Phe | His | Ile | $CH_2-C_6H_5$ | H | 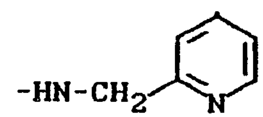 | 0,21 (i) |
| 80 | BOC | Pro | Phe | His | Ile | $CH_2-C_6H_5$ | H | 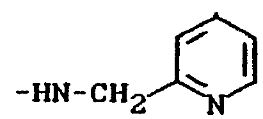 | 0,28 bzw. 0,19 (i) |

Beispiel 81

N-[2-Acetamidomethyl-4-(S)-Boc-L-phenylalanyl-L-histidyl-amino-3-hydroxy-5-phenylpentanoyl]-L-isoleucinmethylester

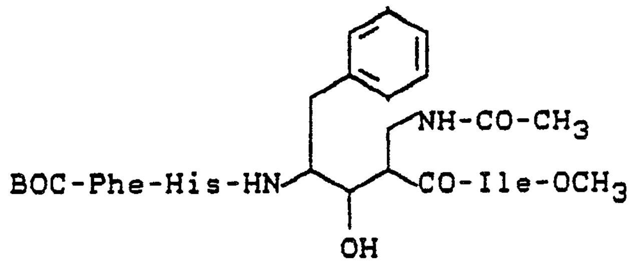

110 mg (0.15 mmol) der Verbindung aus Beispiel 77 in 5 ml Methylenchlorid wurden bei 0° C mit 14 µl (0.15 mmol) Acetanhydrid und 21 µl (0.15 mmol) Triethylamin versetzt und das Gemisch 30 Min. lang bei 0° C gerührt. Die Lösung wurde zweimal mit ges. Kochsalzlösung gewaschen, über Natriumsulfat getrocknet, eingeengt, und das Rohprodukt durch Säulenchromatographie an Kieselgel in 10:1 Methylenchlorid/Methanol gereinigt.
Ausbeute: 88 mg = 74 % d.Th.
$R_F$ (a): 0,27

Beispiel 82

N-[2-Aminomethyl-4-(S)-Boc-L-phenylalanyl-2-histidyl-amino-5-cyclohexyl-3-hydroxypentanoyl]-2-isoleucinmethylester

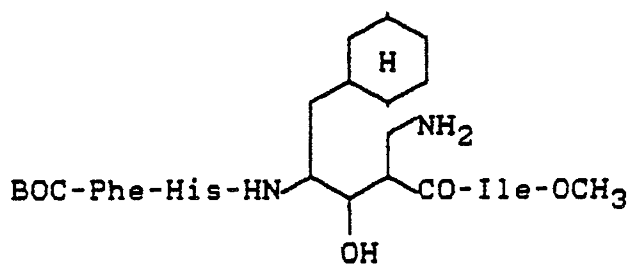

300 mg 10%-iger Pd/C-Katalysator wurden zu einer Lösung von 500 mg (0.59 mmol) der Verbindung aus Beispiel 39 (unpolares Isomer) und 186 mg (2.95 mmol) Ammoniumformiat in 20 ml Methanol gegeben und die Mischung unter Argon eine Stunde lang unter Rückfluß erhitzt. Der Katalysator wurde abfiltriert, das Filtrat eingeengt, der Rückstand in Methylenchlorid aufgenommen, einmal mit halbges. Kochsalzlösung gewaschen, über Natriumsulfat getrocknet, eingeengt, und das Rohprodukt durch Säulenchromatographie an Kieselgel in 9:1:0.1 Methylenchlorid/Methanol/konz. wäßr. Ammoniaklösung gereinigt.
Ausbeute: 259 mg = 58% d. Th.
$R_F$ (i): 0,12 bzw. 0,09
Die in Tabelle 5 aufgeführten Beispiele wurden analog der Vorschrift von Beispiel 82 hergestellt.

Tabelle 5

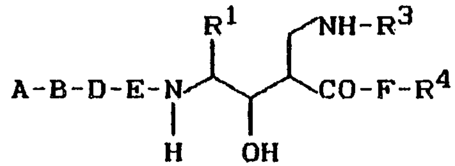

| Beispiel Nr. | A | B | D | E | F | $R^1$ | $R^3$ | $R^4$ | $R_f$ |
|---|---|---|---|---|---|---|---|---|---|
| 83 | BOC | Pro | Phe | His | Ile | $CH_2-C_6H_{11}$ | H | $OCH_3$ | 0,14 (i) |
| 84 | BOC | - | Phe | His | Ile | $CH_2-C_6H_{11}$ | H | 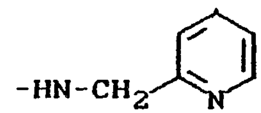 | 0,26 (i) |
| 85 | BOC | Pro | Phe | His | Ile | $CH_2-C_6H_{11}$ | H | 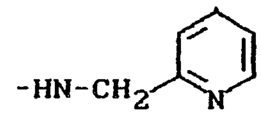 | 0,25 (i) |
| 86 | BOC | - | Phe | His | - | $CH_2-C_6H_{11}$ | H | $-NH-iC_4H_9$ | 0,13 (i) |
| 87 | BOC | - | Phe | His | - | $CH_2-C_6H_{11}$ | H | $-NH-C_2H_4-C_6H_5$ | 0,14 (i) |
| 88 | - | - | 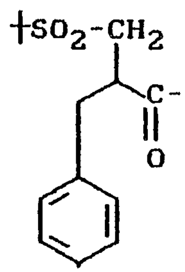 | 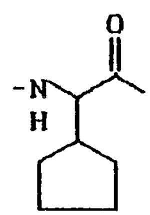 | Ile | $CH_2-C_6H_{11}$ | H | 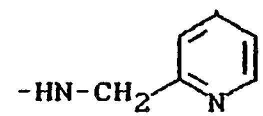 | 0,48 bzw. 0,41 (i) |

Tabelle 5 (Fortsetzung)

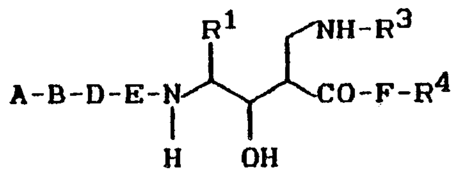

| Beispiel Nr. | A | B | D | E | F | $R^1$ | $R^3$ | $R^4$ | $R_f$ |
|---|---|---|---|---|---|---|---|---|---|
| 89 | Boc | - | Phe | 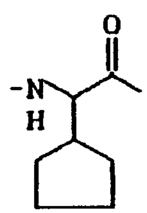 | Ile | $CH_2-C_6H_{11}$ | H | 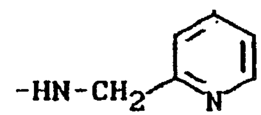 | 0,49 (i) |
| 90 | Boc | - | 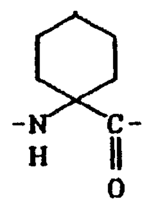 | His | Ile | $CH_2C_6H_{11}$ | H | 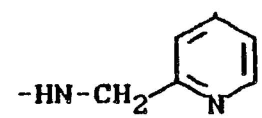 | 0,23 (i) |
| 91 | - | - | 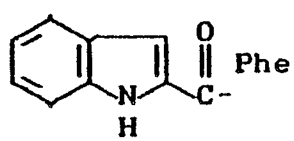 | Phe | Ile | $CH_2-C_6H_{11}$ | H | 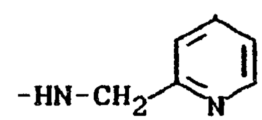 | 0,53 bzw. 0,39 (i) |

Tabelle 5 (Fortsetzung)

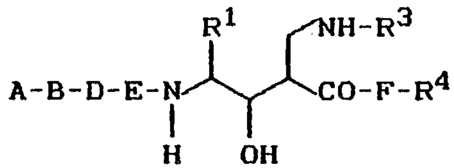

| Beispiel Nr. | A | B | D | E | F | $R^1$ | $R^3$ | $R^4$ | $R_f$ |
|---|---|---|---|---|---|---|---|---|---|
| 92 | - | - | 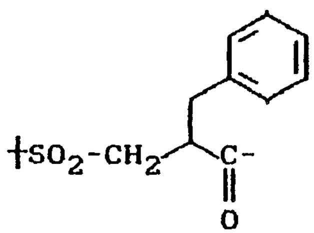 | His | Ile | $CH_2-C_6H_{11}$ | H | 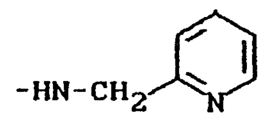 | 0,29 bzw. 0,16 (i) |
| 93 | Boc | - | 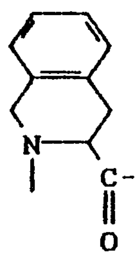 | His | Ile | $CH_2-C_6H_{11}$ | H | 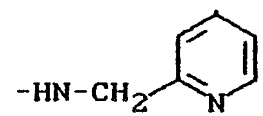 | 0,20 (i) |

Beispiel 94

N-[2-Aminomethyl-4-(S)-Boc-L-phenylalanyl-L-histidyl-amino-3-hydroxy-6-methylheptanoyl]-L-isoleucinmethylester

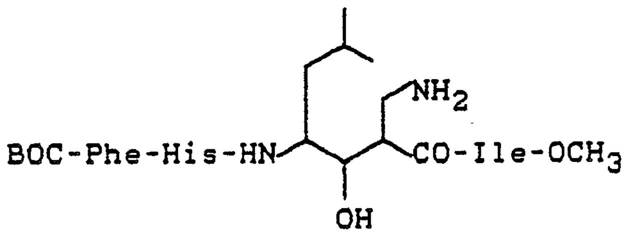

300 mg 10%-iger Pd/C-Katalysator wurden zu einer Lösung von 500 mg (0.62 mmol) der Verbindung aus Beispiel 60 (unpolares Isomer) und 196 mg (3.1 mmol) Ammoniumformiat in 20 ml Methanol gegeben und die Mischung unter Argon eine Stunde lang unter Rückfluß erhitzt. Der Katalysator wurde abfiltriert, das Filtrat eingeengt, der Rückstand in Methylenchlorid aufgenommen, einmal mit halbges. Kochsalzlösung gewaschen, über Natriumsulfat getrocknet, eingeengt, und das Rohprodukt durch Säulenchromatographie an Kieselgel in 9:1:0.1 Methylenchlorid/Methanol/konz. wäßr. Ammoniaklösung gereinigt.

Ausbeute: 336 mg = 75.7% d.Th. (Lyophilisat)

$R_F$ (i): 0,20

Die in Tabelle 6 aufgeführten Beispiele wurden analog der Vorschrift von Beispiel 94 hergestellt.

Tabelle 6

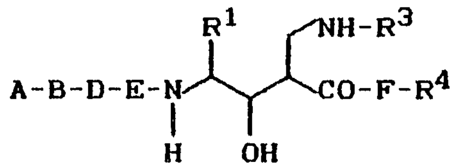

| Beispiel Nr. | A | B | D | E | F | $R^1$ | $R^3$ | $R^4$ | $R_F$ |
|---|---|---|---|---|---|---|---|---|---|
| 95 | BOC | Pro | Phe | His | Ile | $iC_4H_9$ | H | $OCH_3$ | 0,21 bzw. 0,16 (i) |
| 96 | BOC | - | Phe | His | Ile | $iC_4H_9$ | H | 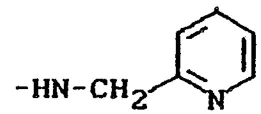 | 0,15 bzw. 0,09 (i) |
| 97 | BOC | Pro | Phe | His | Ile | $iC_4H_9$ | H | 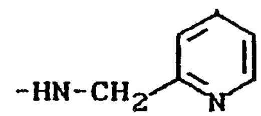 | 0,13 bzw. 0,09 (i) |
| 98 | 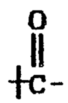 | - | Phe | His | Ile | $iC_4H_9$ | H | 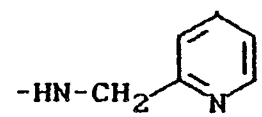 | 0,17 bzw. 0,08 (i) |
| 99 | - | - | 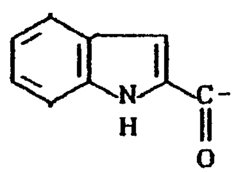 | 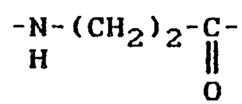 | Ile | $iC_4H_9$ | H | 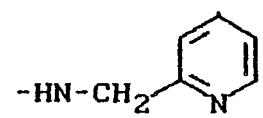 | 0,20 bzw. 0,17 (i) |

<u>Tabelle 6</u> (Fortsetzung)

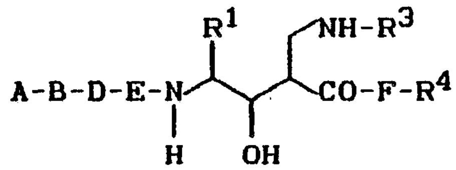

| Beispiel Nr. | A | B | D | E | F | $R^1$ | $R^3$ | $R^4$ | $R_F$ |
|---|---|---|---|---|---|---|---|---|---|
| 100 | - | - | 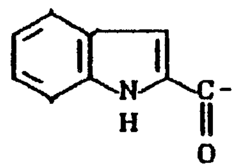 | His | Ile | $iC_4H_9$ | H | $-HN-CH_2-$ 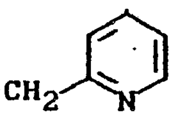 | 0,14 bzw. 0,08 (i) |
| 101 | BOC | - | 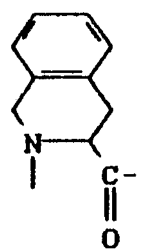 | 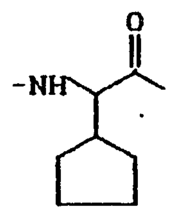 | Ile | $iC_4H_9$ | H | $-HN-CH_2-$ 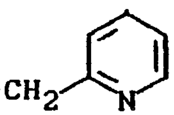 | 0,47 (i) |
| 102 | - | - | 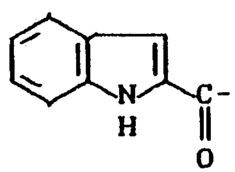 | Phe | Ile | $iC_4H_9$ | H | $-HN-CH_2-$ 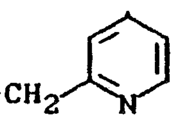 | 0,40 bzw. 0,29 (i) |

Tabelle 6 (Fortsetzung)

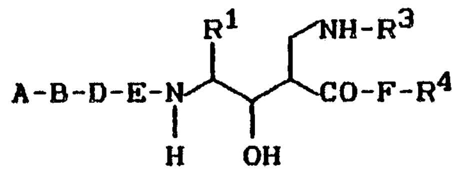

| Beispiel Nr. | A | B | D | E | F | $R^1$ | $R^3$ | $R^4$ | $R_F$ |
|---|---|---|---|---|---|---|---|---|---|
| 103 | BOC | - | Phe | 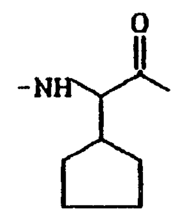 | Ile | $iC_4H_9$ | H | 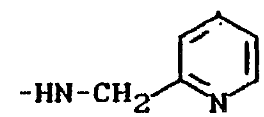 | 0,43 (i) |
| 104 | BOC | - | - | 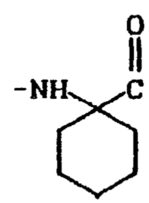 | Ile | $iC_4H_9$ | H | 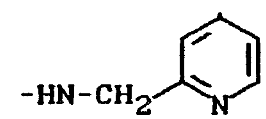 | 0,40 bzw. 0,37 (i) |
| 105 | - | - | - | 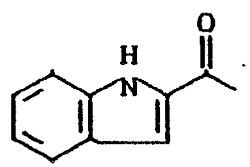 | Ile | $iC_4H_9$ | H | 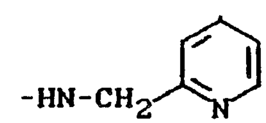 | 0,39 (i) |

Tabelle 6 (Fortsetzung)

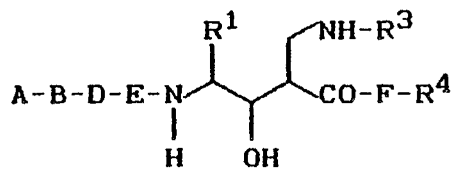

| Beispiel Nr. | A | B | D | E | F | $R^1$ | $R^3$ | $R^4$ | $R_F$ |
|---|---|---|---|---|---|---|---|---|---|
| 106 | 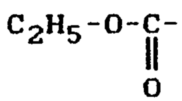 | - | Phe | His | Ile | $iC_4H_9$ | H | 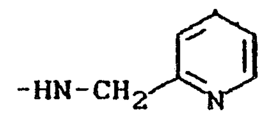 | 0,29 bzw. 0,14 (i) |
| 107 | - | - | 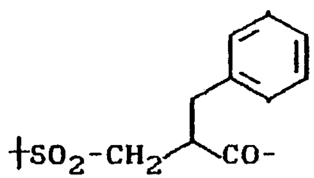 | His | Ile | $iC_4H_9$ | H | 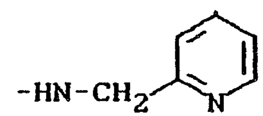 | 0,35 bzw. 0,20 (i) |
| 108 | BOC | - | 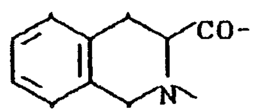 | His | Ile | $iC_4H_9$ | H | 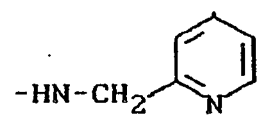 | 0,29 (i) |

Tabelle 6 (Fortsetzung)

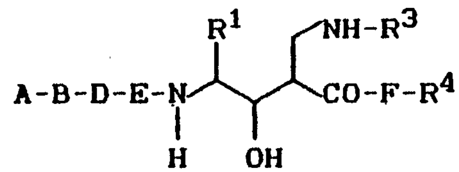

| Beispiel Nr. | A | B | D | E | F | $R^1$ | $R^3$ | $R^4$ | $R_F$ |
|---|---|---|---|---|---|---|---|---|---|
| 109 | BOC | - | 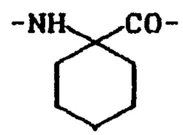 | His | Ile | $iC_4H_9$ | H | 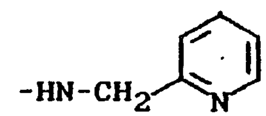 | 0,40 (i) |

**Ansprüche**

1. Aminomethyl-peptide der allgemeinen Formel (I)

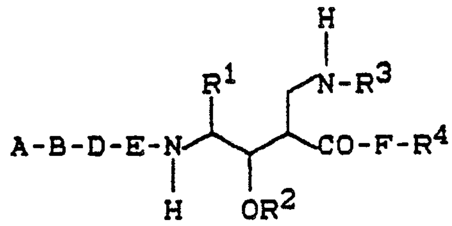

(I)

in welcher

A - Wasserstoff bedeutet oder

- für $C_1$-$C_8$-Alkyl, $C_1$-$C_8$-Alkylsulfonyl, Phenylsulfonyl oder Tolylsulfonyl steht oder

- für eine Gruppe der Formel $COR^5$ oder $COOR^6$ steht

worin

$R^5$ - für geradkettiges oder verzweigtes Alkyl mit bis zu 10 Kohlenstoffatomen steht, das gegebenenfalls durch Hydroxy, Aryl, Amino, Alkylamino mit bis zu 8 Kohlenstoffatomen je Alkylgruppe oder Dialkylamino mit bis zu 8 Kohlenstoffatomen je Alkylgruppe substituiert ist

und

$R^6$ - für geradkettiges oder verzweigtes Alkyl mit bis zu 8 Kohlenstoffatomen steht

oder

A - für eine Aminoschutzgruppe steht

B - für eine direkte Bindung steht oder

- für eine Gruppe der Formel

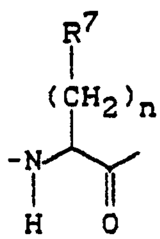 , 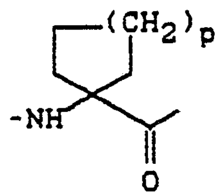 , 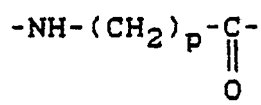

oder 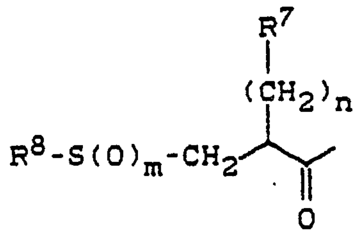 steht

worin

p - eine Zahl 1, 2 oder 3 bedeutet

m - eine Zahl 0, 1 oder 2 bedeutet

n - eine Zahl 0, 1, 2, 3 oder 4 bedeutet

$R^7$ - Wasserstoff, $C_1$-$C_8$-Alkyl, Hydroxymethyl, Hydroxyethyl, Carboxy, $C_1$-$C_8$-Alkoxycarbonyl oder Mercaptomethyl bedeutet oder

- für eine Gruppe der Formel $-CH_2-NH-R^8$ steht

worin

$R^8$ - für Wasserstoff, $C_1$-$C_8$-Alkyl, Phenylsulfonyl, $C_1$-$C_8$-Alkylsulfonyl oder

- für eine Aminoschutzgruppe steht
$R^7$ - Guanidinomethyl, Methylthiomethyl, Halogen, Indolyl, Imidazolyl, Pyridyl, Triazolyl oder Pyrazolyl bedeutet, das gegebenenfalls durch $R^8$ substituiert ist
wobei
$R^8$ die oben angegebene Bedeutung hat oder
- für Cycloalkyl mit 3 bis 8 Kohlenstoffatomen steht
- für Aryl steht, das bis zu 3-fach gleich oder verschieden substituiert sein kann durch $C_1$-$C_6$-Alkyl, $C_1$-$C_6$-Alkoxy, $C_1$-$C_6$-Alkylbenzyloxy, Trifluormethyl, Halogen, Hydroxy, Nitro oder durch eine Gruppe der Formel

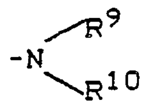

worin
$R^9$ und $R^{10}$ gleich oder verschieden sind und
- für Wasserstoff, $C_1$-$C_8$-Alkyl, $C_1$-$C_6$-Alkylsulfonyl, Aryl, Aralkyl, Tolylsulfonyl, Acetyl, Benzoyl oder
- für eine Aminoschutzgruppe stehen oder
B - für eine Gruppe der Formel

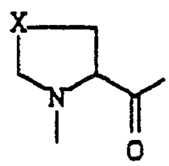 , 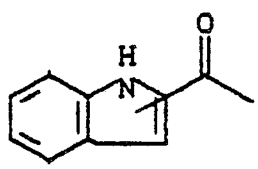 oder 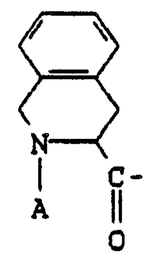 steht

worin
X - für Methylen, Hydroxymethylen, Ethylen, Schwefel oder Sauerstoff steht,
und
A die oben angegebene Bedeutung hat
D - die oben angegebene Bedeutung von B hat und mit dieser gleich oder verschieden ist,
E - die oben angegebene Bedeutung von B hat und mit dieser gleich oder verschieden ist,
F - die oben angegebene Bedeutung von B hat und mit dieser gleich oder verschieden ist,
$R^1$ - für geradkettiges oder verzweigtes Alkyl mit bis zu 10 Kohlenstoffatomen steht, das gegebenenfalls durch Halogen, Hydroxy, Cycloalkyl mit 3 bis 8 Kohlenstoffatomen, Amino, Alkylamino mit bis zu 8 Kohlenstoffatomen, Dialkylamino mit bis zu 8 Kohlenstoffatomen je Alkylgruppe oder Phenyl substituiert ist, welches seinerseits durch $C_1$-$C_8$-Alkyl, Amino, Nitro, Cyano oder Halogen substituiert sein kann oder
- für Aryl mit 6 bis 10 Kohlenstoffatomen steht, das bis zu 4-fach gleich oder verschieden durch $C_1$-$C_6$-Alkyl, $C_1$-$C_6$-Alkoxy, Hydroxy, Cyano, Nitro, Trifluormethyl, Trifluormethoxy, Trifluormethylthio, Phenyl oder durch die Gruppe der Formel

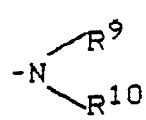

substituiert sein kann
worin
$R^9$ und $R^{10}$ gleich oder verschieden sind und die oben angegebene Bedeutung haben,
$R^2$ - Wasserstoff bedeutet oder
- für geradkettiges oder verzweigtes Alkyl mit bis zu 10 Kohlenwasserstoffatomen steht oder
- für die Gruppe der Formel
$-COR^5$ steht

worin
$R^5$ die oben angegebene Bedeutung hat,
$R^3$ - Wasserstoff bedeutet oder
- für geradkettiges oder verzweigtes Alkyl mit bis zu 10 Kohlenstoffatomen steht, das substituiert sein kann durch Halogen, Hydroxy, Aryl, Aralkyl oder Heteroaryl oder
- für eine Gruppe der Formel
$-COR^5$ steht
worin
$R^5$ die oben angegebene Bedeutung hat
$R^3$ - für Aryl steht, das bis zu 4-fach gleich oder verschieden substituiert sein kann durch Halogen, Hydroxy, Nitro, Cyano $C_1$-$C_8$-Alkoxy, $C_1$-$C_8$-Alkyl oder Amino
$R^4$ - für geradkettiges oder verzweigtes Alkyl mit bis zu 10 Kohlenstoffatomen steht, das gegebenenfalls durch Hydroxy, Cycloalkyl mit 3 bis 8 Kohlenstoffatomen, Halogen, $C_1$-$C_8$-Alkoxy, $C_1$-$C_8$-Alkoxycarbonyl oder Aryl substituiert ist oder
- für $C_1$-$C_8$-Alkoxy steht oder
- für Aryl steht, das bis zu 3-fach gleich oder verschieden durch Halogen, Hydroxy, Nitro, Cyano, Amino oder $C_1$-$C_8$-Alkoxy subtituiert sein kann oder
- für einen Rest
$-HN-R^{11}$ steht
worin
$R^{11}$ - Wasserstoff bedeutet oder
- für geradkettiges oder verzweigtes Alkyl mit bis zu 10 Kohlenstoffatomen steht, das gegebenenfalls durch Hydroxy, Halogen, $C_1$-$C_8$-Alkoxy, $C_1$-$C_8$-Alkoxycarbonyl, Cycloalkyl mit 3 bis 8 Kohlenstoffatomen, Aryl oder Heteroaryl substituiert list oder
- für Cycloalkyl mit 3 bis 8 Kohlenstoffatomen steht oder
- für Phenyl steht, das durch Hydroxy, Halogen, Nitro, Cyano, $C_1$-$C_8$-Alkyl, $C_1$-$C_8$-Alkoxycarbonyl oder durch Amino substituiert sein kann,
und deren physiologisch unbedenklichen Salze.

2. Verbindungen der allgemeinen Formel (I) gemäß Anspruch 1, in welcher
A - Wasserstoff bedeutet oder
- für $C_1$-$C_6$-Alkyl, $C_1$-$C_6$-Alkylsulfonyl, Phenylsulfonyl oder Tolylsulfonyl steht oder
- für eine Gruppe der Formel $COR^5$ oder $COOR^6$ steht
worin
$R^5$ für geradkettiges oder verzweigtes Alkyl mit bis zu 8 Kohlenstoffatomen steht, das gegebenenfalls durch Hydroxy, Phenyl, Amino, Alkylamino mit bis zu 6 Kohlenstoffatomen oder Dialkylamino mit bis zu 6 Kohlenstoffatomen je Alkylgruppe substituiert ist
und
$R^6$ - für geradkettiges oder verzweigtes Alkyl mit bis zu 6 Kohlenstoffatomen steht
oder
A - für eine Aminoschutzgruppe steht
B - für eine direkte Bindung steht oder
- für einen Rest der Formel

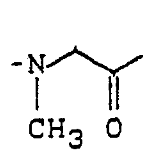 , 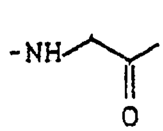 , 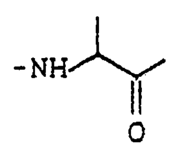 ,

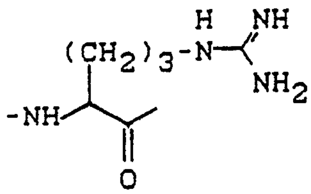 , 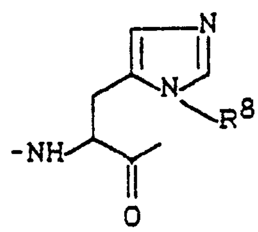 , 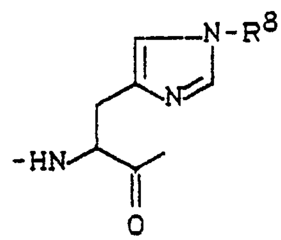

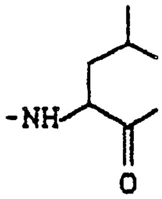 , 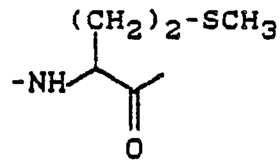 , 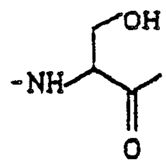

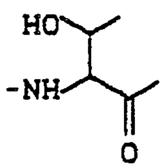 , 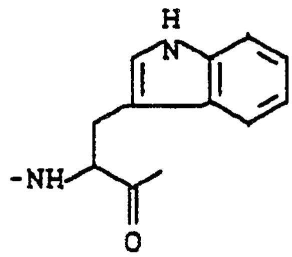 , 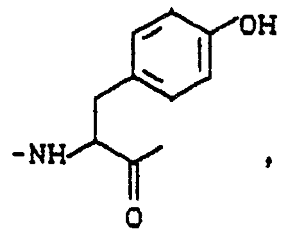 ,

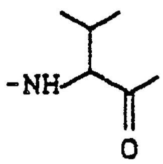 , 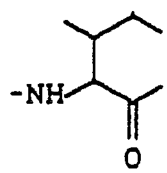 , 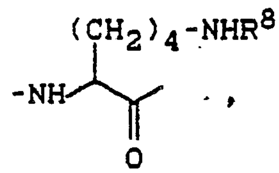 , 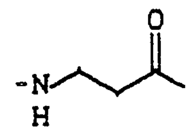 ,

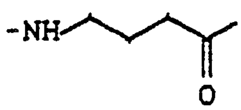 , 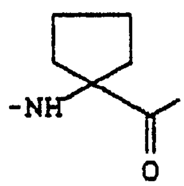 , 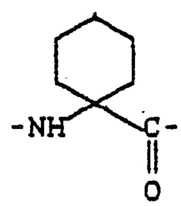 ,

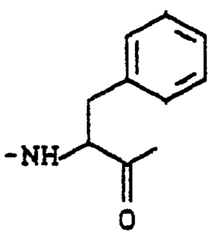 , 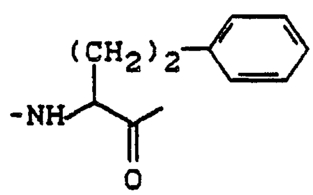 , 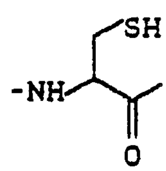 ,

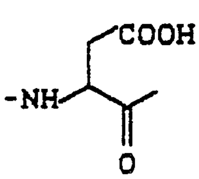 , 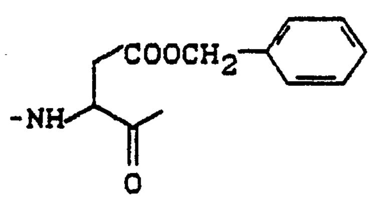 , 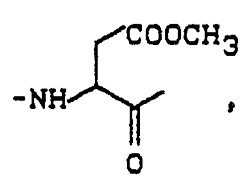 ,

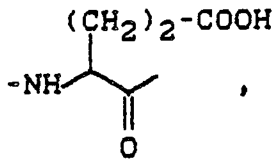 , 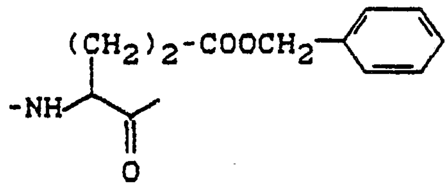 ,

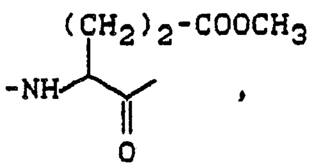 , 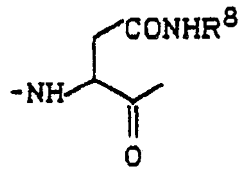 ,

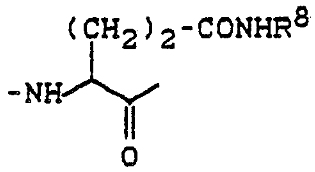 , 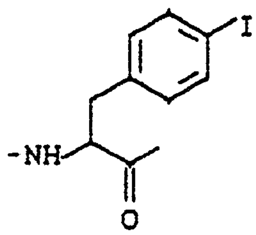 , 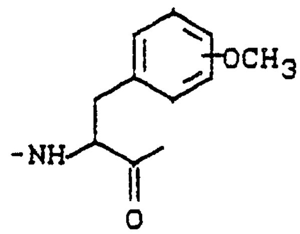 ,

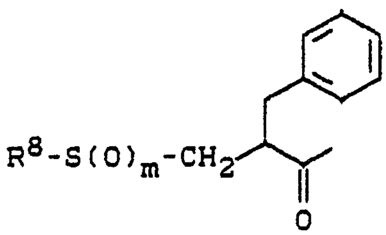 , 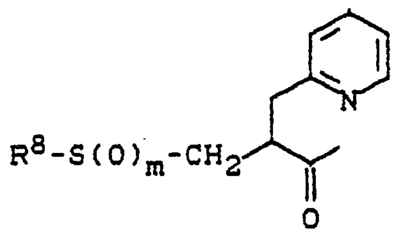 ,

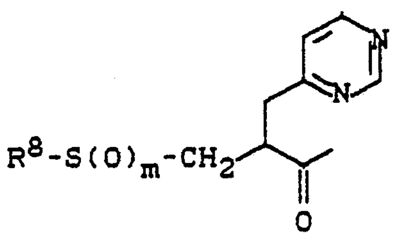 , 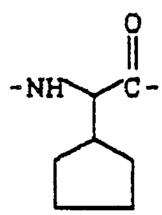 , 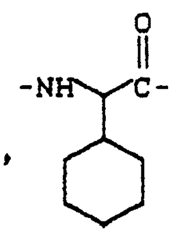

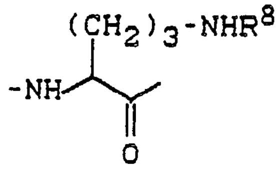, 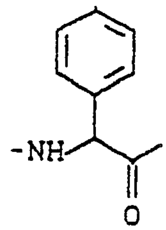, 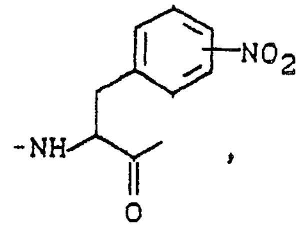,

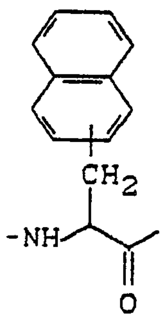, 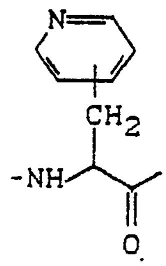, 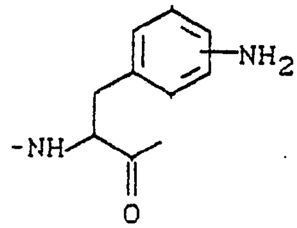

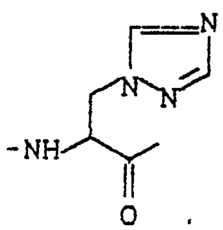 oder 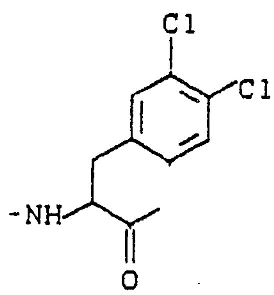

in ihrer D-Form, L-Form oder als D,L-Isomerengemisch, bevorzugt in der L-Form,
worin
m - eine Zahl 0, 1 oder 2 bedeutet
$R^8$ - Wasserstoff bedeutet oder
- für $C_1$-$C_6$-Alkyl, Phenylsulfonyl, $C_1$-$C_4$-Alkylsulfonyl oder
- für eine Aminoschutzgruppe steht,
B - für eine Gruppe der Formel

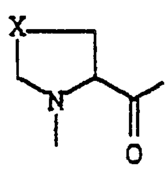, 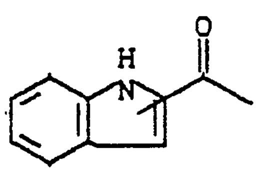 oder 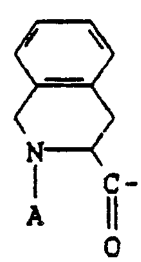 steht

worin
X - Methylen, Schwefel oder Sauerstoff bedeutet
und
A die oben angegebene Bedeutung hat
in ihrer L-Form, D-Form oder als D,L-Isomerengemisch,
D, E und F gleich oder verschieden sind und die gleiche Bedeutung wie B haben und mit dieser gleich oder verschieden sind

$R^1$ - für geradkettiges oder verzweigtes Alkyl mit bis zu 8 Kohlenstoffatomen steht, das gegebenenfalls durch Fluor, Chlor, Brom, Hydroxy, Cycloalkyl mit 3 bis 8 Kohlenstoffatomen, Amino, Alkylamino mit bis zu 6 Kohlenstoffatomen, Dialkylamino mit bis zu 6 Kohlenstoffatomen je Alkylgruppe oder Phenyl substituiert ist, welches seinerseits durch $C_1$-$C_6$-Alkyl, Amino, Nitro, Cyano oder Halogen substituiert sein kann oder
- für Phenyl steht, das bis zu 3-fach gleich oder verschieden durch $C_1$-$C_4$-Alkyl, $C_1$-$C_4$-Alkoxy, Hydroxy, Cyano, Nitro, Trifluormethyl, Trifluormethoxy, Trifluormethylthio, Phenyl oder durch eine Gruppe der Formel

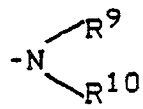

substituiert sein kann
worin
$R^9$ und $R^{10}$ gleich oder verschieden sind
und
- für Wasserstoff, $C_1$-$C_6$-Alkyl, $C_1$-$C_4$-Alkylsulfonyl, Phenyl, Benzyl, Tolylsulfonyl, Acetyl oder Benzoyl stehen oder
- eine Aminoschutzgruppe bedeuten,
$R^2$ - Wasserstoff bedeutet, oder
- für geradkettiges oder verzweigtes Alkyl mit bis zu 8 Kohlenstoffatomen steht oder
- für die Gruppe der Formel $COR^5$ steht,
worin
$R^5$ die oben angegebene Bedeutung hat,
$R^3$ - Wasserstoff bedeutet oder
- für geradkettiges oder verzweigtes Alkyl mit bis zu 8 Kohlenstoffatomen steht, das substituiert sein kann durch Fluor, Chlor, Brom, Hydroxy, Phenyl, Benzyl, Pyridyl oder Pyrimidyl oder
- für eine Gruppe der Formel $COR^5$ steht,
worin
$R^5$ die oben angegebene Bedeutung hat
oder
$R^3$ - für Phenyl steht, das bis zu 3-fach gleich oder verschieden substituiert sein kann durch Fluor, Chlor, Brom, Hydroxy, Nitro, Cyano, $C_1$-$C_4$-Alkoxy, $C_1$-$C_4$-Alkyl oder Amino
$R^4$ - für geradkettiges oder verzweigtes Alkyl mit bis zu 8 Kohlenstoffatomen steht, das gegebenenfalls durch Hydroxy, Cyclopropyl, Cyclopentyl, Cyclohexyl, Fluor, Chlor, Brom, $C_1$-$C_6$-Alkoxy, $C_1$-$C_6$-Alkoxycarbonyl oder Phenyl substituiert ist oder
- für $C_1$-$C_6$-Alkoxy steht oder
- für Phenyl steht, das bis zu 2-fach gleich oder verschieden durch Fluor, Chlor, Brom, Hydroxy, Nitro, Cyano, Amino oder $C_1$-$C_6$-Alkoxy substituiert sein kann oder
- für einen Rest
$-HN-R^{11}$ steht
worin
$R^{11}$ - Wasserstoff bedeutet oder
- für geradkettiges oder verzweigtes Alkyl mit bis zu 8 Kohlenstoffatomen steht, das gegebenenfalls durch Hydroxy, Fluor, Chlor, Brom, $C_1$-$C_6$-Alkoxy, $C_1$-$C_6$-Alkoxycarbonyl, Cyclopropyl, Cyclopentyl, Cyclohexyl, Phenyl, Pyridyl oder Pyrimidyl substituiert ist oder
- für Cyclopropyl, Cyclopentyl oder Cyclohexyl steht oder
- für Phenyl steht, das durch Hydroxy, Fluor, Chlor, Brom, Nitro, Cyano, $C_1$-$C_6$-Alkyl, $C_1$-$C_6$-Alkoxycarbonyl oder durch Amino substituiert sein kann,
und deren physiologisch unbedenklichen Salze.

3. Verbindungen der allgemeinen Formel (I) gemäß Anspruch 1, in welcher
A - Wasserstoff bedeutet oder
- für $C_1$-$C_4$-Alkyl, $C_1$-$C_4$-Alkylsulfonyl, Phenylsulfonyl oder Tolylsulfonyl steht oder
- eine Gruppe der Formel
$-COR^5$ oder $-COOR^6$ steht
worin
$R^5$ - für geradkettiges oder verzweigtes Alkyl mit bis zu 6 Kohlenstoffatomen steht, das gegebenenfalls durch Hydroxy, Phenyl, Amino, Alkylamino mit bis zu 4 Kohlenstoffatomen oder durch Dialkylamino mit bis

zu 4 Kohlenstoffatomen je Alkylgruppe substituiert ist

$R^6$ - für geradkettiges oder verzweigtes Alkyl mit bis zu 4 Kohlenstoffatomen steht oder

A - für eine Aminoschutzgruppe steht,

B - für eine direkte Bindung steht oder

- für Glycyl (Gly), Alanyl (Ala), Arginyl (Arg), Histidyl (His), Leucyl (Leu), Isoleucyl (Ile), Seryl (Ser), Threonyl (Thr), Tryptophyl (Trp), Tyrosyl (Tyr), Valyl (Val), Lysyl (Lys), Asparagyl (Asp), Asparaginamido (Asn), Glutamyl (Glu), Glutaminamido (Gln), Cystyl (Cys), Methionyl (Met), Phenylalanyl (Phe), 2-, 3- oder 4-Nitrophenylalanyl, 2-, 3- oder 4-Aminophenylalanyl oder Pyridylalanyl, gegebenenfalls mit Aminoschutzgruppe,

in ihrer L-Form oder D-Form oder

- für D- oder L-Prolyl (Pro) steht

- für eine Gruppe der Formel

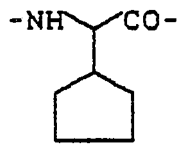 , 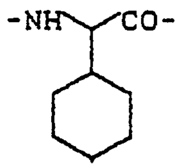 , 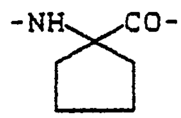 , 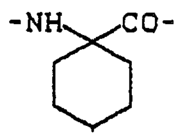 , 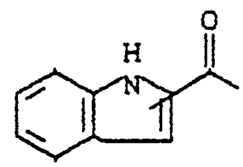 ,

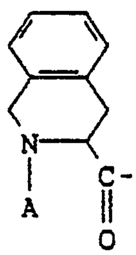 , 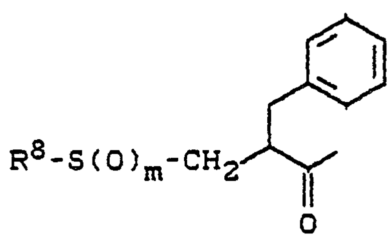 oder 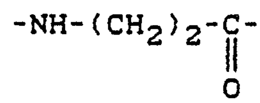 ,

steht

worin

A die oben angegebene Bedeutung hat und

$R^8$ für $C_1$-$C_4$-Alkyl steht

D, E und F gleich oder verschieden sind und die gleiche Bedeutung wie B haben und mit dieser gleich oder verschieden sind,

$R^1$ - für geradkettiges oder verzweigtes Alkyl mit bis zu 6 Kohlenstoffatomen steht, das gegebenenfalls durch Fluor, Chlor, Hydroxy, Cyclopropyl, Cyclopentyl, Cyclohexyl, Amino, Alkylamino mit bis zu 4 Kohlenstoffatomen, Dialkylamino mit bis zu 4 Kohlenstoffatomen je Alkylgruppe, oder Phenyl substituiert ist, welches seinerseits durch $C_1$-$C_3$-Alkyl, Amino, Nitro, Cyano, Fluor oder Chlor substituiert sein kann oder

- für Phenyl steht, das bis zu 2-fach gleich oder verschieden durch $C_1$-$C_3$-Alkyl, $C_1$-$C_2$-Alkoxy, Hydroxy, Cyano, Nitro, Trifluormethyl, Trifluormethoxy, Trifluormethylthio, Phenyl oder durch eine Gruppe der Formel

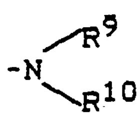

substituiert sein kann

worin

$R^9$ und $R^{10}$ gleich oder verschieden sind und

- für Wasserstoff, $C_1$-$C_3$-Alkyl, $C_1$-$C_3$-Alkylsulfonyl, Phenyl, Benzyl, Tolylsufonyl, Acetyl oder Benzoyl stehen oder

- eine Aminoschutzgruppe bedeuten,

$R^2$ Wasserstoff bedeutet oder

- für geradkettiges oder verzweigtes Alkyl mit bis zu 6 Kohlenstoffatomen steht oder

- für die Gruppe der Formel

$-COR^5$ steht

worin

$R^5$ die oben angegebene Bedeutung hat
$R^3$ - Wasserstoff bedeutet oder
- für geradkettiges oder verzweigtes Alkyl mit bis zu 6 Kohlenstoffatomen steht, das substituiert sein kann durch Fluor, Chlor, Hydroxy, Phenyl, Pyridyl oder Pyrimidyl oder
- für eine Gruppe der Formel
$-COR^5$ steht
worin
$R^5$ die oben angegebene Bedeutung hat oder
$R^3$ - für Phenyl steht, das bis zu 2-fach gleich oder verschieden substituiert sein kann durch Fluor, Chlor, Hydroxy, Nitro, Cyano, $C_1$-$C_2$-Alkoxy, $C_1$-$C_3$-Alkyl oder Amino
$R^4$ - für geradkettiges oder verzweigtes Alkyl mit bis zu 6 Kohlenstoffatomen steht, das gegebenenfalls durch Hydroxy, Cyclopropyl, Cyclopentyl, Cyclohexyl, Fluor, Chlor, $C_1$-$C_4$-Alkoxy, $C_1$-$C_4$-Alkoxycarbonyl oder Phenyl substituiert ist oder
- für $C_1$-$C_4$-Alkoxy oder
- für Phenyl steht, das durch Fluor, Chlor, Hydroxy, Nitro, Cyano, Amino oder $C_1$-$C_4$-Alkoxy substituiert sein kann
oder
- für einen Rest
$H-N-R^{11}$ steht
worin
$R^{11}$ - Wasserstoff bedeutet oder
- für geradkettiges oder verzweigtes Alkyl mit bis zu 6 Kohlenstoffatomen steht, das gegebenenfalls durch Hydroxy, Fluor, Chlor, Brom, $C_1$-$C_4$-Alkoxy, $C_1$-$C_4$-Alkoxycarbonyl, Cyclopropyl, Cyclopentyl, Cyclohexyl, Phenyl, Pyridyl oder Pyrimidyl substituiert ist oder
- für Cyclopropyl, Cyclopentyl oder Cyclohexyl steht oder
- für Phenyl steht, das durch Fluor, Chlor, Brom, Nitro, Cyano, $C_1$-$C_3$-Alkyl, $C_1$-$C_3$-Alkoxycarbonyl oder durch Amino substituiert sein kann,
sowie deren physiologisch unbedenkliche Salze.

4. Verbindungen der allgemeinen Formel (I) gemäß Anspruch 1 zur Verwendung bei der Bekämpfung von Krankheiten.

5. Verfahren zur Herstellung von Verbindungen der allgemeinen Formel (I)

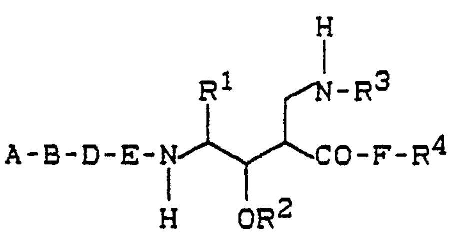

(I)

in welcher
A - Wasserstoff bedeutet oder
- für $C_1$-$C_8$-Alkyl, $C_1$-$C_8$-Alkylsulfonyl, Phenylsulfonyl oder Tolylsulfonyl steht oder
- für eine Gruppe der Formel $COR^5$ oder $COOR^6$ steht
worin
$R^5$ - für geradkettiges oder verzweigtes Alkyl mit bis zu 10 Kohlenstoffatomen steht, das gegebenenfalls durch Hydroxy, Aryl, Amino, Alkylamino mit bis zu 8 Kohlenstoffatomen je Alkylgruppe oder Dialkylamino mit bis zu 8 Kohlenstoffatomen je Alkylgruppe substituiert ist
und
$R^6$ - für geradkettiges oder verzweigtes Alkyl mit bis zu 8 Kohlenstoffatomen steht
oder
A - für eine Aminoschutzgruppe steht
B - für eine direkte Bindung steht oder
- für eine Gruppe der Formel

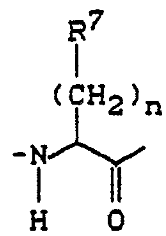 , 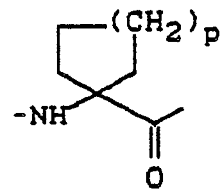 , 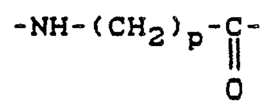

oder 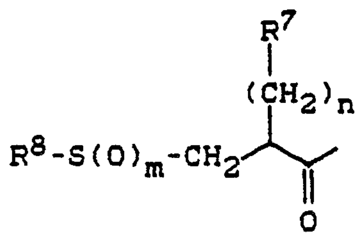 steht

worin

p - eine Zahl 1, 2 oder 3 bedeutet

m - eine Zahl 0, 1 oder 2 bedeutet

n - eine Zahl 0, 1, 2, 3 oder 4 bedeutet

$R^7$ - Wasserstoff, $C_1$-$C_8$-Alkyl, Hydroxymethyl, Hydroxyethyl, Carboxy, $C_1$-$C_8$-Alkoxycarbonyl oder Mercaptomethyl bedeutet oder

- für eine Gruppe der Formel $-CH_2-NH-R^8$ steht

worin

$R^8$ - für Wasserstoff, $C_1$-$C_8$-Alkyl, Phenylsulfonyl, $C_1$-$C_8$-Alkylsulfonyl oder

- für eine Aminoschutzgruppe steht

$R^7$ - Guanidinomethyl, Methylthiomethyl, Halogen, Indolyl, Imidazolyl, Pyridyl, Triazolyl oder Pyrazolyl bedeutet, das gegebenenfalls durch $R^8$ substituiert ist

wobei

$R^8$ die oben angegebene Bedeutung hat oder

- für Cycloalkyl mit 3 bis 8 Kohlenstoffatomen steht

- für Aryl steht, das bis zu 3-fach gleich oder verschieden substituiert sein kann durch $C_1$-$C_6$-Alkyl, $C_1$-$C_6$-Alkoxy, $C_1$-$C_6$-Alkylbenzyloxy, Trifluormethyl, Halogen, Hydroxy, Nitro oder durch eine Gruppe der formel

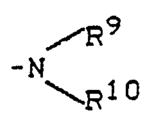

worin

$R^9$ und $R^{10}$ gleich oder verschieden sind und

- für Wasserstoff, $C_1$-$C_8$-Alkyl, $C_1$-$C_6$-Alkylsulfonyl, Aryl, Aralkyl, Tolylsulfonyl, Acetyl, Benzoyl oder

- für eine Aminoschutzgruppe stehen oder

B - für eine Gruppe der Formel

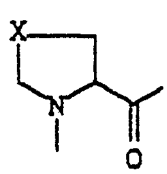 , 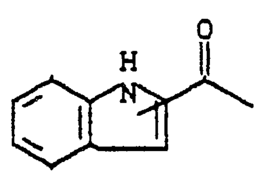 oder 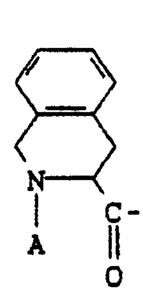 steht

worin
X - für Methylen, Hydroxymethylen, Ethylen, Schwefel oder Sauerstoff steht,
und
A die oben angegebene Bedeutung hat·
D - die oben angegebene Bedeutung von B hat und mit dieser gleich oder verschieden ist,
E - die oben angegebene Bedeutung von B hat und mit dieser gleich oder verschieden ist,
F - die oben angegebene Bedeutung von B hat und mit dieser gleich oder verschieden ist,
$R^1$ - für geradkettiges oder verzweigtes Alkyl mit bis zu 10 Kohlenstoffatomen steht, das gegebenenfalls durch Halogen, Hydroxy, Cycloalkyl mit 3 bis 8 Kohlenstoffatomen, Amino, Alkylamino mit bis zu 8 Kohlenstoffatomen, Dialkylamino mit bis zu 8 Kohlenstoffatomen je Alkylgruppe oder Phenyl substituiert ist, welches seinerseits durch $C_1$-$C_8$-Alkyl, Amino, Nitro, Cyano oder Halogen substituiert sein kann oder
- für Aryl mit 6 bis 10 Kohlenstoffatomen steht, das bis zu 4-fach gleich oder verschieden durch $C_1$-$C_6$-Alkyl, $C_1$-$C_6$-Alkoxy, Hydroxy, Cyano, Nitro,Trifluormethyl, Trifluormethoxy, Trifluormethylthio, Phenyl oder durch die Gruppe der Formel

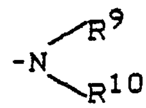

substituiert sein kann
worin
$R^9$ und $R^{10}$ gleich oder verschieden sind und die oben angegebene Bedeutung haben,
$R^2$ - Wasserstoff bedeutet oder
- für geradkettiges oder verzweigtes Alkyl mit bis zu 10 Kohlenwasserstoffatomen steht oder
- für die Gruppe der Formel
$-COR^5$ steht
worin
$R^5$ die oben angegebene Bedeutung hat,
$R^3$ - Wasserstoff bedeutet oder
- für geradkettiges oder verzweigtes Alkyl mit bis zu 10 Kohlenstoffatomen steht, das substituiert sein kann durch Halogen, Hydroxy, Aryl oder Heteroaryl oder
- für eine Gruppe der Formel
$-COR^5$ steht
worin
$R^5$ die oben angegebene Bedeutung hat
$R^3$ - für Aryl steht, das bis zu 4-fach gleich oder verschieden substituiert sein kann durch Halogen, Hydroxy, Nitro, Cyano $C_1$-$C_8$-Alkoxy, $C_1$-$C_8$-Alkyl oder Amino
$R^4$ - für geradkettiges oder verzweigtes Alkyl mit bis zu 10 Kohlenstoffatomen steht, das gegebenenfalls durch Hydroxy, Cycloalkyl mit 3 bis 8 Kohlenstoffatomen, Halogen, $C_1$-$C_8$-Alkoxy, $C_1$-$C_8$-Alkoxycarbonyl oder Aryl substituiert ist oder
- für $C_1$-$C_8$-Alkoxy steht oder
- für Aryl steht, das bis zu 3-fach gleich oder verschieden durch Halogen, Hydroxy, Nitro, Cyano, Amino oder $C_1$-$C_8$-Alkoxy subtituiert sein kann oder
- für einen Rest
$-HN-R^{11}$ steht
worin
$R^{11}$ - Wasserstoff bedeutet oder
- für geradkettiges oder verzweigtes Alkyl mit bis zu 10 Kohlenstoffatomen steht, das gegebenenfalls durch Hydroxy, Halogen, $C_1$-$C_8$-Alkoxy, $C_1$-$C_8$-Alkoxycarbonyl, Cycloalkyl mit 3 bis 8 Kohlenstoffatomen, Aryl oder Heteroaryl substituiert ist oder
- für Cycloalkyl mit 3 bis 8 Kohlenstoffatomen steht oder
- für Phenyl steht, das durch Hydroxy, Halogen, Nitro, Cyano, $C_1$-$C_8$-Alkyl, $C_1$-$C_8$-Alkoxycarbonyl oder durch Amino substituiert sein kann,
und deren physiologisch unbedenklichen Salze,
dadurch gekennzeichnet, daß man Verbindungen der allgemeinen Formel (II)

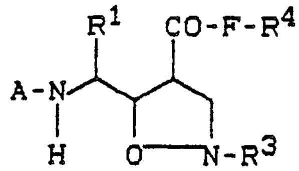

(II)

in welcher
$R^1$, $R^3$, $R^4$, A und F die oben angegebene Bedeutung haben,
zunächst deblockiert, indem die Gruppe A nach üblicher Methode abgespalten wird, und in einem zweiten Schritt mit Verbindungen der allgemeinen Formel (III)
A-B-D-E-OH (III)
in welcher
A, B, D und E die oben angegebene Bedeutung haben,
zu den Verbindungen der allgemeinen Formel (IIa)

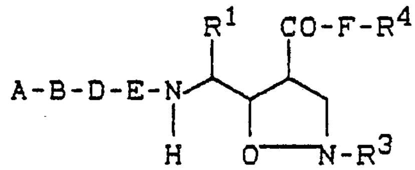

(IIa)

in welcher
A, B, D, E, F, $R^1$, $R^3$ und $R^4$ die oben angegebene Bedeutung haben,
umsetzt,
und die Verbindungen der allgemeinen Formel (IIa) anschließend durch Hydrogenolyse unter Ringöffnung reduziert.

6. Verbindungen der allgemeinen Formel

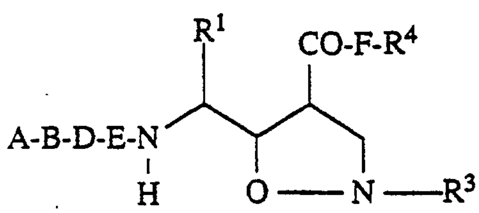

(IIa)

in welcher
A - Wasserstoff bedeutet oder
- für $C_1$-$C_8$-Alkyl, $C_1$-$C_8$-Alkylsulfonyl, Phenylsulfonyl oder Tolylsulfonyl steht oder
- für eine Gruppe der Formel $COR^5$ oder $COOR^6$ steht
worin
$R^5$ - für geradkettiges oder verzweigtes Alkyl mit bis zu 10 Kohlenstoffatomen steht, das gegebenenfalls durch Hydroxy, Aryl, Amino, Alkylamino mit bis zu 8 Kohlen stoffatomen je Alkylgruppe oder Dialkylamino mit bis zu 8 Kohlenstoffatomen je Alkylgruppe substituiert ist
und
$R^6$ - für geradkettiges oder verzweigtes Alkyl mit bis zu 8 Kohlenstoffatomen steht
oder
A - für eine Aminoschutzgruppe steht
B - für eine direkte Bindung steht oder
- für eine Gruppe der Formel

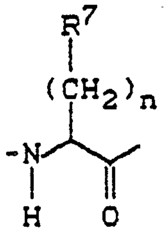 , 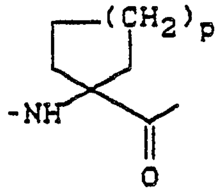 , 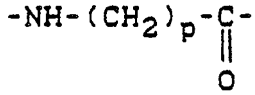

oder 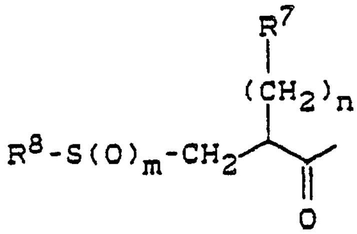 steht

worin

p - eine Zahl 1, 2 oder 3 bedeutet

m - eine Zahl 0, 1 oder 2 bedeutet

n - eine Zahl 0, 1, 2, 3 oder 4 bedeutet

$R^7$ - Wasserstoff, $C_1$-$C_8$-Alkyl, Hydroxymethyl, Hydroxyethyl, Carboxy, $C_1$-$C_8$-Alkoxycarbonyl oder Mercaptomethyl bedeutet oder

- für eine Gruppe der Formel $-CH_2-NH-R^8$ steht

worin

$R^8$ - für Wasserstoff, $C_1$-$C_8$-Alkyl, Phenylsulfonyl, $C_1$-$C_8$-Alkylsulfonyl oder

- für eine Aminoschutzgruppe steht

$R^7$ - Guanidinomethyl, Methylthiomethyl, Halogen, Indolyl, Imidazolyl, Pyridyl, Triazolyl oder Pyrazolyl bedeutet, das gegebenenfalls durch $R^8$ substituiert ist

wobei

$R^8$ die oben angegebene Bedeutung hat oder

- für Cycloalkyl mit 3 bis 8 Kohlenstoffatomen steht

- für Aryl steht, das bis zu 3-fach gleich oder verschieden substituiert sein kann durch $C_1$-$C_6$-Alkyl, $C_1$-$C_6$-Alkoxy, $C_1$-$C_6$-Alkylbenzyloxy, Trifluormethyl, Halogen, Hydroxy, Nitro oder durch eine Gruppe der Formel

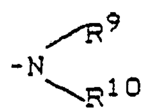

worin

$R^9$ und $R^{10}$ gleich oder verschieden sind und

- für Wasserstoff, $C_1$-$C_8$-Alkyl, $C_1$-$C_6$-Alkylsulfonyl, Aryl, Aralkyl, Tolylsulfonyl, Acetyl, Benzoyl oder

- für eine Aminoschutzgruppe stehen oder

B - für eine Gruppe der Formel

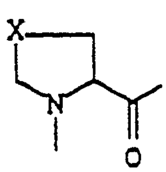 , 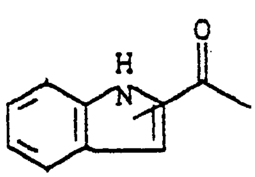 oder 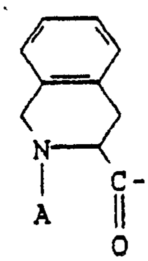 steht

worin
X - für Methylen, Hydroxymethylen, Ethylen, Schwefel oder Sauerstoff steht,
und
A die oben angegebene Bedeutung hat
D - die oben angegebene Bedeutung von B hat und mit dieser gleich oder verschieden ist,
E - die oben angegebene Bedeutung von B hat und mit dieser gleich oder verschieden ist,
F - die oben angegebene Bedeutung von B hat und mit dieser gleich oder verschieden ist,
$R^1$ - für geradkettiges oder verzweigtes Alkyl mit bis zu 10 Kohlenstoffatomen steht, das gegebenenfalls durch Halogen, Hydroxy, Cycloalkyl mit 3 bis 8 Kohlenstoffatomen, Amino, Alkylamino mit bis zu 8 Kohlenstoffatomen, Dialkylamino mit bis zu 8 Kohlenstoffatomen je Alkylgruppe oder Phenyl substituiert ist, welches seinerseits durch $C_1$-$C_8$-Alkyl, Amino, Nitro, Cyano oder Halogen substituiert sein kann oder
- für Aryl mit 6 bis 10 Kohlenstoffatomen steht, das bis zu 4-fach gleich oder verschieden durch $C_1$-$C_6$-Alkyl, $C_1$-$C_6$-Alkoxy, Hydroxy, Cyano, Nitro,Trifluormethyl, Trifluormethoxy, Trifluormethylthio, Phenyl oder durch die Gruppe der Formel

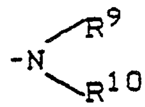

substituiert sein kann
worin
$R^9$ und $R^{10}$ gleich oder verschieden sind und die oben angegebene Bedeutung haben,
$R^2$ - Wasserstoff bedeutet oder
- für geradkettiges oder verzweigtes Alkyl mit bis zu 10 Kohlenwasserstoffatomen steht oder
- für die Gruppe der Formel
$-COR^5$ steht
worin
$R^5$ die oben angegebene Bedeutung hat,
$R^3$ - Wasserstoff bedeutet oder
- für geradkettiges oder verzweigtes Alkyl mit bis zu 10 Kohlenstoffatomen steht, das substituiert sein kann durch Halogen, Hydroxy, Aryl, Aralkyl oder Heteroaryl oder
- für eine Gruppe der Formel
$-COR^5$ steht
worin
$R^5$ die oben angegebene Bedeutung hat
$R^3$ - für Aryl steht, das bis zu 4-fach gleich oder verschieden substituiert sein kann durch Halogen, Hydroxy, Nitro, Cyano $C_1$-$C_8$-Alkoxy, $C_1$-$C_8$-Alkyl oder Amino
$R^4$ - für geradkettiges oder verzweigtes Alkyl mit bis zu 10 Kohlenstoffatomen steht, das gegebenenfalls durch Hydroxy, Cycloalkyl mit 3 bis 8 Kohlenstoffatomen, Halogen, $C_1$-$C_8$-Alkoxy, $C_1$-$C_8$-Alkoxycarbonyl oder Aryl substituiert ist oder
- für $C_1$-$C_8$-Alkoxy steht oder
- für Aryl steht, das bis zu 3-fach gleich oder verschieden durch Halogen, Hydroxy, Nitro, Cyano, Amino oder $C_1$-$C_8$-Alkoxy subtituiert sein kann oder
- für einen Rest
$-HN-R^{11}$ steht
worin
$R^{11}$ - Wasserstoff bedeutet oder
- für geradkettiges oder verzweigtes Alkyl mit bis zu 10 Kohlenstoffatomen steht, das gegebenenfalls durch Hydroxy, Halogen, $C_1$-$C_8$-Alkoxy, $C_1$-$C_8$-Alkoxycarbonyl, Cycloalkyl mit 3 bis 8 Kohlenstoffatomen, Aryl oder Heteroaryl substituiert ist oder
- für Cycloalkyl mit 3 bis 8 Kohlenstoffatomen steht oder
- für Phenyl steht, das durch Hydroxy, Halogen, Nitro, Cyano, $C_1$-$C_8$-Alkyl, $C_1$-$C_8$-Alkoxycarbonyl oder durch Amino substituiert sein kann,
und deren physiologisch unbedenklichen Salze.

7. Verfahren zur Herstellung von Verbindungen der allgemeinen Formel (II) gemäß Anspruch 6, dadurch gekennzeichnet, daß man Verbindungen der allgemeinen Formel (IIb)

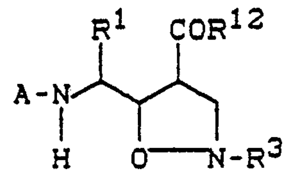

(IIb)

in welcher
A, $R^1$ und $R^3$ die oben angegebene Bedeutung haben und
$R^{12}$ - für $C_1$-$C_4$-Alkoxy steht,
zunächst nach üblichen Methoden verseift und anschießend mit Verbindungen der allgemeinen Formel (IV)
H-F-$R^4$ (IV)
in welcher
F und $R^4$ die oben angegebene Bedeutung haben,
umsetzt.

8. Verbindungen der allgemeinen Formel (IIb)

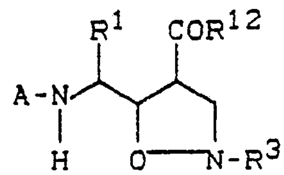

(IIb)

in welcher
A, $R^1$ und $R^3$ die im Anspruch 1 angegebene Bedeutung haben und
$R^{12}$ - für $C_1$-$C_4$-Alkoxy steht.

9. Verfahren zur Herstellung von Verbindungen der allgemeinen Formel (IIb) gemäß Anspruch 8, dadurch gekennzeichnet, daß man Verbindungen der allgemeinen Formel (V)

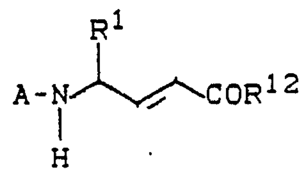

(V)

in welcher
A, $R^1$ und $R^{12}$ die oben angegebene Bedeutung haben,
mit Verbindungen der allgemeinen Formel (VI)

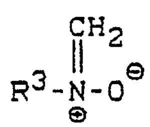

(VI)

in welcher
$R^3$ die oben angegebene Bedeutung hat, in einer Nitron-Cycloadditionsreaktion umsetzt.

10. Arzneimittel enthaltend mindestens eine Verbindung der allgemeinen Formel (I) gemäß Anspruch 1.

11. Verfahren zur Herstellung von Arzneimitteln, dadurch gekennzeichnet, daß man Verbindungen der allgemeinen Formel (I) gemäß Anspruch 1 gegebenenfalls unter Verwendung von üblichen Hilfs- und Trägerstoffen in eine geeignete Applikationsform überführt.

12. Verwendung von Verbindungen der allgemeinen Formel (I) gemäß Anspruch 1 bei der Herstellung von Arzneimitteln mit kreislaufbeeinflussender Wirkung.

13. Verwendung von Verbindungen der allgemeinen Formel (I) gemäß Anspruch 1 zur Herstellung von Arzneimitteln zur Behandlung von Bluthochdruck und Herzinsuffizienz.

14. Verwendung von Verbindungen der allgemeinen Formel (I) gemäß Anspruch 1 bei der Bekämpfung von Kreislauferkrankungen.